Europäisches Patentamt

(19) European Patent Office

Office européen des brevets

(11) Publication number: 0 117 063

A1

(12) **EUROPEAN PATENT APPLICATION**

(21) Application number: 84300312.0

(22) Date of filing: 18.01.84

(51) Int. Cl.³: **C 12 N 15/00**
C 12 P 21/04, C 12 P 21/00
C 12 N 7/00, C 07 C 103/52
A 61 K 39/23

(30) Priority: 19.01.83 US 459203
06.01.84 US 567968

(43) Date of publication of application:
29.08.84 Bulletin 84/35

(84) Designated Contracting States:
AT BE CH DE FR GB IT LI LU NL SE

(71) Applicant: Amgen
1900 Oak Terrace Lane
Thousand Oaks California 91320(US)

(72) Inventor: Fox, Gary M.
11 Dorrit Court
Newbury Park California 91320(US)

(72) Inventor: Hu, Sylvia S.
3256 Peppermint Street
Newbury Park California 91320(US)

(74) Representative: Brown, John David et al,
FORRESTER & BOEHMERT Widenmayerstrasse 4/I
D-8000 München 22(DE)

(54) Methods and materials for development of parvovirus vaccine.

(57) Novel immunologically active polypeptides for use in anti-parvovirus vaccines are provided through structural analysis and characterization of the parvovirus genome. In a preferred embodiment, microbial expression of polypeptides is secured through use of DNA vectors comprising DNA sequences duplicative of porcine parvovirus (PPV) genomic DNA. Microbially expressed polypeptides as well as synthetic replicas of polypeptides coded for by DNA sequences of the invention exhibit immunological activity in, e.g., plate binding assays.

EP 0 117 063 A1

Croydon Printing Company Ltd.

"METHODS AND MATERIALS FOR DEVELOPMENT
OF PARVOVIRUS VACCINE"

## BACKGROUND

The present invention relates generally to mammalian parvoviruses and more particularly to immunologically active polypeptides useful in development of vaccinal immunity against parvovirus infection.

Parvoviruses, members of the family Parvoviridae, are among the simplest viruses known to infect eucaryotic cells. Of the three genera, the Parvovirus and Adeno-associated-virus (AAV) subgroups cover the viruses of vertebrates, whereas the genus Densovirus (densonucleosis virus, DNV) comprises arthropod agents. Vertebrae hosts for parvovirus infection include cattle, pigs, dogs, cats, rats, humans and certain avian species.

The vertebrate parvoviruses are divided into two genera or subgroups on the basis of their requirement for helper viruses. Viruses of the largest subgroup, the genus Parvovirus, are usually referred to as the "nondefective" or autonomous parvoviruses. These are capable of productive replication without the aid of a helper virus in the vast majority of host cells studied to date. On the other hand, all members of the Adeno-associated-virus subgroup are "defective" and are entirely dependent upon adenovirus coinfection for their own replication in all systems examined so far. The viruses are strikingly similar in physiochemical properties growth characteristics in vitro, and, in general, pathogenic potential. They are among the smallest known DNA viruses, being spherical, nonenveloped particles 20-25 nm in diameter. In the electron microscope these particles appear to possess an icosahedral symmetry and comprise 32 morphological capsomers. The infectious particle contains the genome, which is a predominantly single-stranded, linear DNA molecule with a molecular weight of $1.35-1.70 \times 10^6$. This DNA accounts for about 25% of the total virion mass; the balance is protein, with no detectable RNA, carbohydrate, or lipid. The viruses are extremely stable with regard to heat, freezing and thawing, lipid solvents, desiccation, detergents, and even low concentrations of chaotropic agents.

Nearly all the vertebrate viruses show hemagglutinating activity. They are mostly antigenically distinct, as measured by hemagglutinin inhibition and complement fixation, apart from the cross-reacting groups. Within these cross-reacting groups, individual viruses can be distinguished by the species of red blood cell they will agglutinate, by host range, and in some cases by pathogenic potential. See, generally, "Replication of Mammalian Parvoviruses", Ward and Tattersall, eds., Cold spring Harbor Laboratory (1978).

Of particular interest to the background of the present invention are a number of publications specifically directed to porcine parvovirus (PPV), an autonomous parvovirus which constitues a major cause of reproductive failure in swine throughout much of the United States and in many major swine producing regions of the world. See, generally, Joo, et al., Arch. Virol., 51, 123 (1976); Mengeling, et al. Am.J.Vet.Res., 37, 1393 (1976); Johnson, et al. Australian Vet. Jour., 52, 80 (1976); and Mengeling, J.Am.Vet.Med.Assoc., 172, 1291 (1978). Symptoms of infection by PPV in adult pigs are usually subclinical. Infection of pregnant dams in

- 3 -

the first half of gestation commonly results in
embryonic death and fetal mummification.  The primary
manifestations of disease are failure to farrow, prolonged gestation and increased farrowing interval,
decrease in litter size, farrowing of stillborn and mummified fetuses, resorption of fetuses into the uterine
lining and neonatal death or decreased vigor.  See,
Cutlip, et al. Am.J.Vet.Res., 36, 1751 (1975).

A number of studies have been performed
relating to the effects of vaccinal and passive immunity
on infection of pigs with PPV.  See, Paul, et al.
Am.J.Vet.Res., 41, 1368 (1980) and references cited
therein.  Inactivated virus vaccines have been developed
and are reputed to provide some degree of protection
against PPV-induced reproductive failure.  See,
Fujisaki, et al., Nat'l. Inst. Anim. Health Quarterly
(Tokyo) 18, 184 (1978); Joo, et al. Aust. Vet. Jour.,
53, 550 (1977); Mengeling, et al., Am.J.Vet.Res., 40,
204 (1979); and Suzuki, et al., Nat'l. Inst. Anim.
Health Quarterly (Tokyo) 16, 81 (1976).  More recently,
modified live PPV virus vaccines and bivalent vaccines
(including pseudorabies immunogens) have been evaluated
for their potential in the control of reproductive
failure in swine.  See, Paul, et al., Am.J.Vet.Res., 41,
2008 (1980) and Mengeling, et al., Am.J.Vet.Res., 42,
600 (1981).

While, as indicated above, some substantial
efforts have been directed toward development of particulate, live or killed parvovirus vaccines, there have
been no reports of use of subunit vaccines in development of immune responses protective against parvovirus
infection.  Indeed, it was only recently reported that
porcine parvovirus structural proteins have been isolated and determined to have immunogenic potentials
independent of their association with viral particles.
More specifically at a meeting of the American Society for Virology conducted at Cornell University on August 2 through 4, 1982, H. S. Joo orally reported the findings of recent research at the University of Minnesota concerning the characterization and immunogenicity of porcine parvovirus structural proteins passaged in swine fetuses. SDS-PAGE isolation and V-8 protease treatment of viral proteins was said to have indicated the presence of three structurally similar proteins, of 90 kd, 65 kd and 60 kd molecular weights. These were said to be capable of stimulating the production of anti-PPV antibodies in rabbits. Written results of this work were later reported in Molitor, et al., J.Virol., 45, 842-854 (1983), verifying that antisera raised against each protein had some ability to neutralize virus activity.

Some structural studies of autonomous replicating parvoviruses have been conducted. See, Astell, et al., Cell, 17, 691 (1979); Rhode, et al. J. Virol., 41, 990 (1982); Revie, P.N.A.S., 76, 5539 (1979); Paradiso, et al., J.Gen.Virol., 62, 113 (1982); and Rhode, J. Virol., 21, 694 (1977), which has not involved investigation of the structure of the PPV genome nor the relationship between its structure and the structure of viral proteins.

More recent studies of the parvovirus genome have been reported by Rhode, et al., J.Virol., 45, 173-184 (1983) and Astell, et al. Nuc.Acids.Res., 11, 999-1018 (1983). Both studies dealt with rodent parvovirus, designated H-1 and MVM, respectively. Rhode, et al. determined the nucleotide sequence of H-1, correlating it to known structural characteristics of parvovirus capsid proteins, VP1 and VP2' and a non-capsid virus protein, NCVP1. The H-1 genome was determined to contain 5176 nucleotides with 2 large open reading frames headed or preceded by a start codon. Graphically depicted structures of the mRNAs of the viral proteins showed that NCVP1 was in the left most reading frame, and slightly overlapped the start positions of the VP1 and VP2' proteins. While the second open reading frame in the right half of the genome was assigned to VP1 and VP2', no detailed arrangement of the coding sequences was identified. VP1 was shown to start just left of the start site for VP2'. Both VP1 and 2' were illustrated to terminate at the same point on the mRNA map.

Astell, et al.(1983) determined the 5081 nucleotide sequence of the genome of Minute Virus of Mice (MVM), and observed open reading frames in the genome similar to those reported by Rhode, et al. for H-1. Preliminary experiments indicated that 3 viral capsid proteins VP1 (83,000 daltons), VP2 (64,000 daltons) and VP3 (61,000) are encoded by the right half of the genome, while a noncapsid polypeptide of 85,000 daltons is encoded at least in part by the left half of the genome. The mRNA maps in this study indicate that the VP1 message begins at the left most portion of the genome, is interrupted by a large intron, then continues for the length of the genome. VP2 is indicated as wholly contained by the map of VP1, the message starting to the left of a small center intron and continuing to the right side of the map.

Also of interest to the background of the present invention are recent efforts directed toward the use of recombinant methods and materials in the development of subunit viral vaccine materials. A typical example of this type of research effort may be found in Rutter, et al., European Patent Application No. 0062574, published October 13, 1982. To date, however, recombinant methods have not been brought to bear in the sequencing, characterization, or manipulation of the parvovirus genome in a manner permitting microbial expression of immunologically active polypeptides which are wholly or partially duplicative of parvovirus protein sequences.

## BRIEF SUMMARY

The present invention provides immunologically active polypeptides useful in the development of vaccinal immunity against parvovirus infection, illustratively, porcine parvovirus (PPV) infection. Polypeptides of the invention generally comprise amino acid sequences which duplicate or substantially duplicate sequences putatively extant in the major protein constituents of the parvovirus virion. Presently preferred polypeptides of the invention include products of microbial expression of novel, vector-borne, DNA sequences which are wholly or partially duplicative of DNA sequences extant in a parvovirus genome. Especially preferred microbially expressed polypeptides of the invention include those comprising series of hydrophilic amino acids which are duplicative of those in the porcine parvovirus protein envelope.

Illustratively provided by the invention is plasmid pSS17 which principally comprises pBR322 DNA and includes an approximately 780 base pair DNA insert duplicative of a DNA sequence extant in the double stranded replicative form (RF) DNA of porcine parvovirus. The cloned PPV DNA insert in pSS17 is operatively associated with the promoter/regulator and initial protein coding region of the E. coli tryptophan synthetase E gene, allowing for inducible expression in a selected host cell of a fusion polypeptide of PPV and E. coli origins.

Other illustrative microbial expression systems provided by the present invention include plasmid p/H6, in which a fragment of pSS17 is placed into operative association with the temperature-sensitive copy control region of a "run-away" expression vector pCFM414, allowing for greater quantities of the "fusion polypeptide" to be expressed in a selected host cell.

Another plasmid S/S3 is provided which includes as approximately 350 base pair DNA insert duplicative of a DNA sequence extant in porcine parvovirus doublestranded RF DNA in association with a pBR322-derived sequence containing a trp promoter and four amino acids of γ interferon, allowing for inducible expression of a γ interferon/PPV fusion polypeptide in a selected host cell.

A third PPV fragment of approximately 475 base pairs is expressed as a fusion polypeptide with a portion of the β galactosidase polypeptide in plasmid pE/H2.5. transformed into a selected host cell. pE/H2.5 is also principally composed of pBR322 DNA sequences including the promoter/regulator and initial protein coding region of the E. coli tryptophan synthetase E gene.

The present invention also encompasses presenty preferred synthetic peptides having amino acid sequences which are wholly or partially duplicative of amino acid sequences of parvovirus. An illustrative desirable synthetic peptide sequence according to the present invention is

NH$_2$-Asn-Leu-Ala-Lys-Lys-Lys-Ala-Lys-
Gly-Thr-Ser-Asn-Thr-Asn-Ser-COOH.

Immunologically active polypeptides of the invention, including microbially-expressed polypeptides, fusion polypeptides and synthetic polypeptides, should provide the synthesis of parvovirus-specific antibodies in host animals to whom the polypeptides are administered and the antibody responses so generated are expected to provide protection against subsequent parvovirus infection, especially porcine parvovirus infection. Illustrations of the probable antigenicity and immunogenicity of the polypeptides of the present invention are provided by in vitro plate binding assays and plaque reduction (neutralizing) assays. Provided by the present invention, therefore, are vaccine compositions comprising immunologically effective amounts of one or more polypeptides of the invention in combination with immunologically acceptable diluents, adjuvants or carriers.

Polypeptides of the invention are also suitable for use as reagent constituents in assays for quantitative detection of parvovirus antigens or parvovirus-associated antibodies in fluid samples. DNA sequences of the invention may, in a like manner, be employed as probes in DNA/DNA hybridization procedures for the detection of complementary parvovirus DNA sequences in samples of various origins.

Other aspects and advantages of the invention will become apparent upon consideration of the following detailed description thereof wherein:

FIGURE 1 is a restriction map of the PPV genome; and

FIGURE 2 illustrates manipulations involved in the manufacture of plasmid pSS17 according to the invention.

## DETAILED DESCRIPTION

The novel polypeptide products and processes which provide an illustration of the present invention are described in the following examples which are directed to procedures involved in the isolation, and characterization of DNA and proteinaceous components of a representative parvovirus, porcine parvovirus (PPV), and procedures relating to securing expression by microbial host cells (including bacteria, yeast and mammalian cells in culture) of PPV polypeptides, and assaying the polypeptides for antigenicity and immunogenicity.

The following example relates to growth and isolation of PPV in culture.

## EXAMPLE 1

Porcine parvovirus (NADL-2 virulent strain obtained from the National Animal Disease Center, U.S.D.A., Ames, Iowa) are propagated in a suitable continuous culture of porcine cells (e.g., PK-15, A.T.C.C. No. CCL-33), propagated in Dulbecco modified Eagle's medium supplemented with 5% fetal calf serum. The following example summarizes the results of isolation and characterization of PPV protein constituents.

## EXAMPLE 2

Lysates of PPV infected cells were labelled in vivo by incorporation to $^{35}$S-methionine in the culture medium. Labelled PPV virions released were then purified by pelleting and CsCl equilibrium sedimentation. Analysis on SDS-PAGE revealed three major polypeptide constituents having respective molecular weights of 90,000, 65,000 and 60,000 daltons. The relative abundance of these three proteins were determined to be 3%, 80 to 90% and 7%, respectively, of the virion mass. V-8 protease digests of each of the three purified proteins provided cleavage patterns which were essentially identical, indicating that the sequences of the smaller proteins are contained in the larger ones. All three proteins were found to be reactive with neutralizing serum obtained from a convalescent pig recovered from PPV infection in a plate binding assay. Attempts to sequence the three proteins using a gas phase protein microsequenator were unsuccessful, indicating that all three PPV proteins have modified or blocked amino termini.

The following example relates to isolation of PPV DNA and development of a restriction endonuclease digestion map for the PPV genome.

EXAMPLE 3

The infectious virion of PPV propagated according to Example 1 was found to have a buoyant density of 1.375 g/c.c. and could be purified by banding in CsCl gradients. The genome of PPV could be labelled with $^3$H-thymidine in vivo and extracted from the virion with phenol. Gel electrophoresis and electron microscopic analysis revealed the genomic DNA of PPV to be a linear, mostly single-stranded DNA molecule with molecular weight of $1.76 \times 10^6$ daltons or 5.0 kilobases. However, about 6% of the PPV DNA is resistant to S1-nuclease digestion, and probably represents the two non-identical, self-complementary structures at the ends of the genome, as found in other nondefective parvoviruses.

DNA from PPV infected cells was prepared following the standard Hirt extraction method. See, Hirt, J.Mol.Biol., 26, 365 (1967). When the DNA from the supernatant fractions was analyzed by agarose gel electrophoresis, two bands corresponding to duplex monomeric (MD, $3 \times 10^6$ daltons) and dimeric (DD, $6.7 \times 10^6$ daltons) genome lengths were observed. Upon heat denaturation and quick cooling, 33% of the MD molecules regenerate the duplex MD, indicating that these MD molecules must have the two complementary strands linked covalently. The other 66% MD molecules, however, convert to single-stranded genome length DNA (SS) under this treatment, indicating that they have "open" ends. Thus the MD population is composed of two types of molecules with either "fold-back" ends or "extended" ends.

The DD molecules, on the other hand, completely converted to MD and SS after heating and quenching,

- 11 -

0117063

suggesting the existence of specific single-stranded nicks near the middle of the molecules.

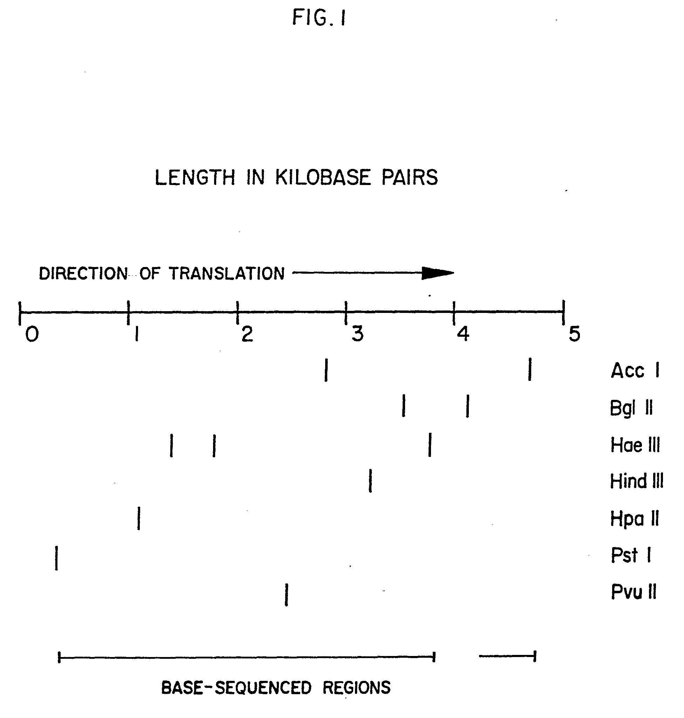

After isolating the MD and DD forms of PPV from preparative gels, a restriction endonuclease cleavage map for PPV was constructed and is illustrated in Figure 1. Briefly summarized, the 5 kilobase PPV monomer replicative form was digested with a number of restriction enzymes and several double digest combinations to produce the restriction cleavage map shown. The enzymes Sall, HpaI, SmaI and BamHI do not cleave PPV DNA. The direction of translation as determined by hybridization studies and the base sequence is shown by the arrow.

The left end of the MD DNA frequently resolved into doublet bands upon cleavage with certain restriction enzymes, a phenomenon that has also been observed for other nondefective parvoviruses. It has been proposed that the slow-migrating band in the doublet represents the "extended" form and the fast-migrating band represents the "snap-back" configuration as described above. The right-hand end, Figure 1, is homogeneous and always gave a single-band upon cleavage. The self-priming mechanism known for the complementary-strand synthesis of parvoviruses suggests that the MD molecules with the "snap-back" configuration when cleaved with restriction enzymes, should generate a fragment with the two strands linked covalently, and this fragment should contain the 3'-OH terminus of the original viral DNA strand.

Based on this assumption, it is appropriate to orient the map with respect to the polarity of the viral DNA as shown in Figure 1 with the left end corresponding to the 3'-terminus of the viral DNA.

Other evidence which supports this orientation is provided by analysis of the transcriptional orientation of PPV. Poly A-containing RNAs from PPV-infected cells, were isolated, fractionated and selected to provide a low molecular weight population which is enriched in the 3' portion of the transcripts. Radioactively labeled cDNA probes were synthesized using oligo (dT) primer and reverse transcriptase. These probes were hybridized to the Southern transfers of various restriction enzyme digests of PPV DNA. The results showed that the right end (Fig. 1) fragments hybridized most strongly with the probes whereas the left end fragments failed to hybridize, indicating that the right end contains sequences complementary to the 3' end of the RNA transcripts. Since PPV mRNAs have polarities opposite to the viral DNA, it can be deduced that the left end corresponds to the 3' terminus of the viral DNA.

- Restriction endonuclease analysis of the PPV duplex dimer (DD) DNA intermediate revealed that the dimeric form is constructed by two MD molecules joined in a tail-to-tail configuration with the 3' ends of the viral strands covalently linked to the complementary strands. It was observed that upon storage the PPV dimer spontaneously converts to duplex monomer MD, indicating that there are staggered, specific single-stranded nicks on the oppoisite strands at unit genome length, i.e., in the middle of the dimer, and that the base-pairing which holds the two units together is rather unstable.

The following example relates to DNA sequence analysis of cloned PPV genomic fragments.

EXAMPLE 4

Fragments derived from the 5 kb monomer replicative form were cloned into either M13 phage or pBR322. These clones fell into two main groups: (1) random clones produced by the cleavage of the 5 kb molecule (or a portion of it) with AluI, RsaI, or Sau3A followed by ligation into appropriately cleaved Ml3mp8; and (2) specific clones generated using restriction sites whose positions have been mapped in Figure 1. Most of these were asymmetric double digests which were cloned into both Ml3mp8 and Ml3mp9 so that their orientation relative to the restriction map was also known. In addition, the 3.3 kb Pstl/EcoRl fragment extending from position 0.3 to 3.6, Figure 1, was cloned into pBR322.

After isolation, the position and orientation of some of the larger random clones was determined by restriction analysis. Since Ml3 phage DNA is single stranded, hybridization of these clones to virion DNA (known to be the (-) strand) established the relationship of the restriction map to the mRNA coding strand as shown in Figure 1. This conclusion was verified by subsequent sequence analysis as described below.

Many of the random clones and all of the specifically constructed clones described above were base sequenced using the Ml3/dideoxy method. The data from overlapping clones were assembled to yield the entire nucleotide sequence from 0.3 to 3.85 kb as illustrated in Table I below, as well as the sequence from about 4.25 to 4.7 kb, illustrated in Table II below.

TABLE I

Translation of DNA PPV

```
   AlaAlaTrpGlnArgGluThrLeuThrArgLysArgTyrOC LysLeuProThrGlyPheLysIleMetLeuLys
   CysSerMetAlaAlaGlyAsnThrTyrSerGluGluValLeuLysAlaThrAsnTrpLeuGlnAspAsnAlaGln
   LeuGlnHisGlySerGlyLysHisLeuLeuGlyArgGlyThrLysSerTyrGlnLeuAlaSerArgOC CysSer
   CTGCAGCATGGCAGCGGGAAACACTTACTCGGAAGAGGTACTAAAAGCTACCAACTGGCTTCAAGATAATGCTCA
   GACGTCGTACCGTCGCCCTTTGTGAATGAGCCTTCTCCATGATTTTCGATGGTTGACCGAAGTTCTATTACGAGT
   GlnLeuMetAlaAlaProPheValOC GluSerSerThrSerPheAlaValLeuGlnSerOP SerLeuAlaOP
     CysCysProLeuProPheCysLysSerProLeuProValLeuLeuAM TrpSerAlaGluLeuTyrHisGluPhe
   AlaAlaHisCysArgSerValSerValArgPheLeuTyrAM PheSerGlyValProLysLeuIleIleSerLeu

   LysLysHisSerLeuMetTyrLeuLysHisLysLysSerIleOC MetGluLysLysLeuLeuGlyIleThrThr
   LysGluAlaPheSerTyrValPheLysThrGlnLysValAsnLeuAsnGlyLysGluIleAlaTrpAsnAsnTyr
   LysArgSerIleLeuLeuCysIleOC AsnThrLysSerGlnSerLysTrpLysArgAsnCysLeuGluOC Leu
   AAAAGAAGCATTCTCTTATGTATTTAAAACACAAAAAGTCAATCTAAATGGAAAAGAAATTGCTTGGAATAACTA
   TTTTCTTCGTAAGAGAATACATAAATTTTGTGTTTTTCAGTTAGATTTACCTTTTCTTTAACGAACCTTATTGAT
   PheSerAlaAsnGluOC ThrAsnLeuValCysPheThrLeuArgPheProPheSerIleAlaGlnPheLeuAM
     LeuLeuMetArgLysHisIleOC PheValPheLeuOP AspLeuHisPheLeuPheGlnLysSerTyrSerCys
   PhePheCysGluArgIleTyrLysPheCysLeuPheAspIleAM IleSerPhePheAsnSerProIleValVal

   ThrLysIleGlnGlnMetArgLysOP OC ThrTyrLysGluGluGlnLysHisHisGlyThrArgGlnGlnThr
   AsnLysAspThrThrAspAlaGluMetIleAsnLeuGlnArgGlyAlaGluThrSerTrpAspGlnAlaThrAsp
   GlnGlnArgTyrAsnArgCysGlyAsnAspLysProThrLysArgSerArgAsnIleMetGlyProGlyAsnArg
   CAACAAAGATACAACAGATGCGGAAATGATAAACCTACAAAGAGGAGCAGAAACATCATGGGACCAGGCAACAGA
   GTTGTTTCTATGTTGTCTACGCCTTTACTATTTGGATGTTTCTCCTCGTCTTTGTAGTACCCTGGTCCGTTGTCT
   LeuLeuSerValValSerAlaSerIleIlePheArgCysLeuProAlaSerValAspHisSerTrpAlaValSer
     CysLeuTyrLeuLeuHisProPheSerLeuGlyValPheLeuLeuLeuPheMetMetProGlyProLeuLeuCys
   ValPheIleCysCysIleArgPheHisTyrValAM LeuSerSerCysPheCysOP ProValLeuCysCysVal
```

TABLE I (cont'd.)

```
    TrpAsnGlyAsnGlnLysSerThrAlaSerGlnAsnGlyLysTyrOP PheLeuThrLeuLeuLeuLysAsnVal
    MetGluTrpGluSerGluIleAspSerLeuThrLysArgGlnValLeuIlePheAspSerLeuValLysLysCys
    HisGlyMetGlyIleArgAsnArgGlnProHisLysThrAlaSerThrAspPheOP LeuSerCysOC LysMet
226 CATGGAATGGGAATCAGAAATCGACAGCCTCACAAAACGGCAAGTACTGATTTTTGACTCTCTTGTTAAAAAATG
    GTACCTTACCCTTAGTCTTTAGCTGTCGGAGTGTTTTGCCGTTCATGACTAAAAACTGAGAGAACAATTTTTTAC
    MetSerHisSerAspSerIleSerLeuArgValPheArgCysThrSerIleLysSerGluArgThrLeuPheHis
       ProIleProIleLeuPheArgCysGlyOP LeuValAlaLeuValSerLysGlnSerGluGlnOC PheIleAsp
    HisPheProPheOP PheAspValAlaGluCysPheProLeuTyrGlnAsnLysValArgLysAsnPhePheThr

    SerLeuLysValTyrCysLysArgThrOC ValGlnValThrAlaThrGlySerTyrSerMetAsnMetValLys
    LeuPheGluGlyIleLeuGlnLysAsnLeuSerProSerAspCysTyrTrpPheLeuGlnHisGluHisGlyGln
    SerLeuOP ArgTyrIleAlaLysGluProLysSerLysOP LeuLeuLeuValLeuThrAlaOP ThrTrpSer
301 TCTCTTTGAAGGTATATTGCAAAAGAACCTAAGTCCAAGTGACTGCTACTGGTTCTTACAGCATGAACATGGTCA
    AGAGAAACTTCCATATAACGTTTTCTTGGATTCAGGTTCACTGACGATGACCAAGAATGTCGTACTTGTACCAGT
    ArgLysSerProIleAsnCysPhePheArgLeuGlyLeuSerGlnAM GlnAsnLysCysCysSerCysProOP
       ArgGlnLeuTyrIleAlaPheSerGlyLeuAspLeuHisSerSerSerThrArgValAlaHisValHisAspLeu
    GluLysPheThrTyrGlnLeuLeuValAM ThrTrpThrValAlaValProGluOC LeuMetPheMetThrLeu

    IleLeuAlaIleThrAlaMetTyrTyrAM ValGluLysAlaTyrAsnLysGlnTrpGluAsnGlyTyrGluAsn
    AspThrGlyTyrHisCysHisValLeuLeuGlyGlyLysGlyLeuGlnGlnAlaMetGlyLysTrpLeuArgLys
    ArgTyrTrpLeuSerLeuProCysThrThrArgTrpLysArgLeuThrThrSerAsnGlyLysMetValThrLys
376 AGATACTGGCTATCACTGCCATGTACTACTAGGTGGAAAAGGCTTACAACAAGCAATGGGAAAATGGTTACGAAA
    TCTATGACCGATAGTGACGGTACATGATGATCCACCTTTTCCGAATGTTGTTCGTTACCCTTTTACCAATGCTTT
    SerValProAM OP GlnTrpThrSerSerProProPheProLysCysCysAlaIleProPheHisAsnArgPhe
       TyrGlnSerAspSerGlyHisValValLeuHisPheLeuSerValValLeuLeuProPheIleThrValPheVal
    IleSerAlaIleValAlaMetTyrAM AM ThrSerPheAlaOC LeuLeuCysHisSerPheProOC SerPhe
```

TABLE I (cont'd.)

AsnOC ThrIleTyrGlyValAspGlyOP IleIleAsnAlaLysTyrLeuOC HisGlnLeuLysGluOC Asn
GlnLeuAsnAsnLeuTrpSerArgTrpLeuAsnAsnGlnCysLysValProLeuThrProValGluArgIleLys
ThrIleLysGlnPheMetGluAM MetValGluOC SerMetGlnSerThrSerAsnThrSerOP LysAsnLys
451 ACAATTAAACAATTTATGGAGTAGATGGTTGAATAATCAATGCAAAGTACCTCTAACACCAGTTGAAAGAATAAA
TGTTAATTTGTTAAATACCTCATCTACCAACTTATTAGTTACGTTTCATGGAGATTGTGGTCAACTTTCTTATTT
CysAsnPheLeuLysHisLeuLeuHisAsnPheLeuOP HisLeuThrGlyArgValGlyThrSerLeuIlePhe
IleLeuCysAsnIleSerTyrIleThrSerTyrAspIleCysLeuValGluLeuValLeuGlnPhePheLeuIle
LeuOC ValIleOC ProThrSerProGlnIleIleLeuAlaPheTyrArgAM CysTrpAsnPheSerTyrPhe

OC GlyAsnAM GlnArgMetValSerGlyTyrArgTyrOC ProThrLeuThrAsnLysLeuLysAsnAsnIle
LeuArgGluLeuAlaGluAspGlyGluTrpValSerLeuLeuThrTyrThrHisLysGlnThrLysLysGlnTyr
IleLysGlyIleSerArgGlyTrpOP ValGlyIleAlaThrAsnLeuHisSerGlnThrAsnOC LysThrIle
526 ATTAAGGGAATTAGCAGAGGATGGTGAGTGGGTATCGCTACTAACCTACACTCACAAACAAACTAAAAAACAATA
TAATTCCCTTAATCGTCTCCTACCACTCACCCATAGCGATGATTGGATGTGAGTGTTTGTTTGATTTTTTGTTAT
AsnLeuSerAsnAlaSerSerProSerHisThrAspSerSerValAM ValOP LeuCysValLeuPheCysTyr
LeuProIleLeuLeuProHisHisThrProIleAlaValLeuArgCysGluCysValPheAM PheValIleTyr
OC ProPheOC CysLeuIleThrLeuProTyrArgAM AM GlyValSerValPheLeuSerPhePheLeuIle

GlnLysOP LeuIleLeuGluGluIleOP LeuLeuThrThrSerOC IleLysLysGluArgGlnLeuLysGluSer
ThrLysMetThrHisPheGlyAsnMetIleAlaTyrTyrPheLeuAsnLysLysArgLysThrThrGluArgGlu
TyrLysAsnAspSerPheTrpLysTyrAspCysLeuLeuLeuProLysOC LysLysLysAspAsnOP LysArg
601 TACAAAAATGACTCATTTTGGAAATATGATTGCTTACTACTTCCTAAATAAAAAAAGAAAGACAACTGAAAGAGA
ATGTTTTTACTGAGTAAAACCTTTATACTAACGAATGATGAAGGATTTATTTTTTTCTTCTGTTGACTTTCTCT
ValPheIleValOP LysProPheIleIleAlaOC AM LysArgPheLeuPheLeuPheValValSerLeuSer
LeuPheSerGluAsnGlnPheTyrSerGlnLysSerSerGlyLeuTyrPhePhePheSerLeuGlnPheLeuAla
CysPheHisSerMetLysSerIleHisAsnSerValValGluAM IlePhePheSerLeuCysSerPheSerLeu

TABLE I (cont'd.)

MetAspIleIleSerAlaGlnIleLeuAlaSerOP GlnIleSerOC LysLysAlaArgAspThrAM SerVal
HisGlyTyrTyrLeuSerSerAspSerGlyPheMetThrAsnPheLeuLysGluGlyGluArgHisLeuValSer
AlaTrpIleLeuSerGlnLeuArgPheTrpLeuHisAspLysPheLeuLysArgArgArgGluThrLeuSerGln
GCATGGATATTATCTCAGCTCAGATTCTGGCTTCATGACAAATTTCTTAAAAGAAGGCGAGAGACACTTAGTCAG
CGTACCTATAATAGAGTCGAGTCTAAGACCGAAGTACTGTTTAAAGAATTTTCTTCCGCTCTCTGTGAATCAGTC
CysProTyrOC ArgLeuGluSerGluProLysMetValPheLysLysPheSerProSerLeuCysLysThrLeu
HisIleAsnAspOP SerLeuAsnGlnSerOP SerLeuAsnArgLeuLeuLeuArgSerValSerLeuOP Asp
MetSerIleIleGluAlaOP IleArgAlaGluHisCysIleGluOC PhePheAlaLeuSerValOC AspThr

ThrTyrLeuLeuLysGlnIleAsnLeuLysLeuTrpLysGlnArgLeuLeuGlnLeuArgLysPheProGluAla
HisLeuPheThrGluAlaAsnLysProGluThrValGluThrThrValThrThrAlaGlnGluValProArgGly
SerProIleTyrOP SerLysOC ThrOP AsnCysGlyAsnAsnGlyTyrTyrSerSerGlySerSerProArg
TCACCTATTTACTGAAGCAAATAAACCTGAAACTGTGGAAACAACGGTTACTACAGCTCAGGAAGTTCCCCGAGG
AGTGGATAAATGACTTCGTTTATTTGGACTTTGACACCTTTGTTGCCAATGATGTCGAGTCCTTCAAGGGGCTCC
OP ArgAsnValSerAlaPheLeuGlySerValThrSerValValThrValValAlaOP SerThrGlyArgPro
GlyIleOC GlnLeuLeuTyrValGlnPheGlnProPheLeuProOC AM LeuGluProLeuGluGlyLeuCys
ValAM LysSerPheCysIlePheArgPheSerHisPheCysArgAsnSerCysSerLeuPheAsnGlySerAla

GluTyrLysGlnLysLysLysOC AlaOC AsnAlaGlnOC GluThrTrpLeuIleLysAspValLeuAlaAM
ArgIleGlnThrLysLysGluValSerIleLysCysThrIleArgAspLeuValAsnLysArgCysThrSerIle
GlnAsnThrAsnLysLysArgSerLysHisLysMetHisAsnLysArgLeuGlyOC OC LysMetTyrAM His
CAGAATACAAACAAAAAAGAAGTAAGCATAAAATGCACAATAAGAGACTTGGTTAATAAAAGATGTACTAGCAT
GTCTTATGTTTGTTTTTTTCTTCATTCGTATTTTACGTGTTATTCTCTGAACCAATTATTTTCTACATGATCGTA
LeuIleCysValPhePheSerThrLeuMetPheHisValIleLeuSerLysThrLeuLeuLeuHisValLeuMet
PheValPheLeuPheLeuLeuLeuCysLeuIleCysLeuLeuLeuSerProOC TyrPheIleTyrAM CysLeu
SerTyrLeuCysPhePhePheTyrAlaTyrPheAlaCysTyrSerValGlnAsnIlePheSerThrSerAlaTyr

TABLE I (cont'd.)

LysAlaGlyValOP GlnIleGlnThrValIleAM LysOP TrpLeuLysProGluGluLysIleOC SerLys
GluGlyTrpSerMetThrAspProAspSerTyrIleGluMetMetAlaGlnThrGlyGlyGluAsnLeuIleLys
ArgArgLeuGluTyrAspArgSerArgGlnLeuTyrArgAsnAspGlySerAsnArgArgArgLysPheAsnGln
AGAAGGCTGGAGTATGACAGATCCAGACAGTTATATAGAAATGATGGCTCAAACCGGAGGAGAAAATTTAATCAA
TCTTCCGACCTCATACTGTCTAGGTCTGTCAATATATCTTTACTACCGAGTTTGGCCTCCTCTTTTAAATTAGTT
SerProGlnLeuIleValSerGlySerLeuOC IleSerIleIleAlaOP ValProProSerPheLysIleLeu
LeuSerSerTyrSerLeuAspLeuCysAsnTyrLeuPheSerProGluPheArgLeuLeuPheAsnLeuOP Phe
PheAlaProThrHisCysIleTrpValThrIleTyrPheHisHisSerLeuGlySerSerPheIleOC AspPhe

IleHisAM LysOC GlnLeuLeuLeuAM GlnGluGlnLysGlnHisMetThrOC TyrLeuLysArgGlnAsn
AsnThrLeuGluIleThrThrLeuThrLeuAlaArgThrLysThrAlaTyrAspLeuIleLeuGluLysAlaLys
LysTyrThrArgAsnAsnAsnSerTyrSerSerLysAsnLysAsnSerIleOP LeuAsnThrOP LysGlyLys
AAATACACTAGAAATAACAACTCTTACTCTAGCAAGAACAAAAACAGCATATGACTTAATACTTGAAAAGGCAAA
TTTATGTGATCTTTATTGTTGAGAATGAGATCGTTCTTGTTTTTGTCGTATACTGAATTATGAACTTTTCCGTTT
PheValSerSerIleValValArgValArgAlaLeuValPheValAlaTyrSerLysIleSerSerPheAlaPhe
TyrValLeuPheLeuLeuGluOC GluLeuLeuPheLeuPheLeuMetHisSerLeuValGlnPheProLeuVal
IleCysAM PheTyrCysSerLysSerAM CysSerCysPheCysCysIleValOC TyrLysPheLeuCysPhe

GlnAlaCysTyrGlnHisLeuIleLeuAlaIleGlnGluHisValLysTyrSerAlaCysThrIleGlyThrThr
ProSerMetLeuProThrPheAsnIleSerAsnThrArgThrCysLysIlePheSerMetHisAsnTrpAsnTyr
ThrLysHisAlaThrAsnIleOC TyrAM GlnTyrLysAsnMetOC AsnIleGlnHisAlaGlnLeuGluLeu
ACCAAGCATGCTACCAACATTTAATATTAGCAATACAAGAACATGTAAAATATTCAGCATGCACAATTGGAACTA
TGGTTCGTACGATGGTTGTAAATTATAATCGTTATGTTCTTGTACATTTTATAAGTCGTACGTGTTAACCTTGAT
GlyLeuMetSerGlyValAsnLeuIleLeuLeuValLeuValHisLeuIleAsnLeuMetCysLeuGlnPheAM
LeuCysAlaValLeuMetOC TyrOC CysTyrLeuPheMetTyrPheIleOP CysAlaCysAsnSerSerCys
TrpAlaHisAM TrpCysLysIleAsnAlaIleCysSerCysThrPheTyrGluAlaHisValIleProValVal

- 18 -

0117063

TABLE I (cont'd.)

LeuLysSerAlaMetLeuOC LeuValTyrOP AsnArgGlnGlyGlyLysArgAsnThrIleLeuPheHisVal
IleLysValCysHisAlaIleThrCysValLeuLysGlnThrArgArgLysLysLysTyrAsnSerIleSerCys
HisOC SerLeuProCysTyrAsnLeuCysThrGluThrAspLysGluGluLysGluIleGlnPheTyrPheMet
CATTAAAGTCTGCCATGCTATAACTTGTGTACTGAAACAGACAAGGAGGAAAAAGAAATACAATTCTATTTCATG
GTAATTTCAGACGGTACGATATTGAACACATGACTTTGTCTGTTCCTCCTTTTTCTTTATGTTAAGATAAAGTAC
MetLeuThrGlnTrpAlaIleValGlnThrSerPheCysValLeuLeuPhePhePheTyrLeuGluIleGluHis
OC LeuArgGlyHisAM LeuLysHisValSerValSerLeuSerSerPheSerIleCysAsnAM LysMetAsp
AsnPheAspAlaMetSerTyrSerThrTyrGlnPheLeuCysProProPheLeuPheValIleArgAsnOP Thr

MetGlyGlnHisGlnGlnGluLysValOC LeuLeuAsnThrLeuGlnThrAM LeuValMetLeuValAlaThr
HisGlyProAlaSerThrGlyLysSerIleIleAlaGlnHisIleAlaAsnLeuValGlyAsnValGlyCysTyr
SerTrpAlaSerIleAsnArgLysLysTyrAsnCysSerThrHisCysLysLeuSerTrpOC CysTrpLeuLeu
TCATGGGCCAGCATCAACAGGAAAAAGTATAATTGCTCAACACATTGCAAACTTAGTTGGTAATGTTGGTTGCTA
AGTACCCGGTCGTAGTTGTCCTTTTTCATATTAACGAGTTGTGTAACGTTTGAATCAACCATTACAACCAACGAT
OP ProGlyAlaAspValProPheLeuIleIleAlaOP CysMetAlaPheLysThrProLeuThrProGlnAM
HisAlaLeuMetLeuLeuPhePheTyrLeuGlnGluValCysGlnLeuSerLeuGlnTyrHisGlnAsnSerCys
MetProTrpCysOP CysSerPheThrTyrAsnSerLeuValAsnCysValOC AsnThrIleAsnThrAlaVal

MetGlnProMetOP ThrPheHisLeuMetThrValGlnIleLysThrOC TyrGlyLeuLysLysGlnGluThr
AsnAlaAlaAsnValAsnPheProPheAsnAspCysThrAsnLysAsnLeuIleTrpIleGluGluAlaGlyAsn
GlnCysSerGlnCysGluLeuSerIleOC OP LeuTyrLysOC LysLeuAsnMetAspOP ArgSerArgLys
CAATGCAGCCAATGTGAACTTTCCATTTAATGACTGTACAAATAAAAACTTAATATGGATTGAAGAAGCAGGAAA
GTTACGTCGGTTACACTTGAAAGGTAAATTACTGACATGTTTATTTTTGAATTATACCTAACTTCTTCGTCCTTT
LeuAlaAlaLeuThrPheLysGlyAsnLeuSerGlnValPheLeuPheLysIleHisIleSerSerAlaProPhe
HisLeuTrpHisSerSerGluMetOC HisSerTyrLeuTyrPheSerLeuIleSerGlnLeuLeuLeuPheSer
IleCysGlyIleHisValLysTrpLysIleValThrCysIlePheValOC TyrProAsnPhePheCysSerVal

0117063

TABLE I (cont'd.)

SerLeuThrLysOC ThrAsnSerLysProTyrValGlnValLysGlnLeuGluLeuThrLysLysValLysGlu
PheSerAsnGlnValAsnGlnPheLysAlaIleCysSerGlyGlnThrIleArgIleAspGlnLysGlyLysGly
LeuLeuOC ProSerLysProIleGlnSerHisMetPheArgSerAsnAsnAM AsnOP ProLysArgOC Arg
CTTCTCTAACCAAGTAAACCAATTCAAAGCCATATGTTCAGGTCAAACAATTAGAATTGACCAAAAAGGTAAAGG
GAAGAGATTGGTTCATTTGGTTAAGTTTCGGTATACAAGTCCAGTTTGTTAATCTTAACTGGTTTTTCCATTTCC
LysGluLeuTrpThrPheTrpAsnLeuAlaMetHisGluProOP ValIleLeuIleSerTrpPheProLeuPro
ArgAM GlyLeuLeuGlyIleOP LeuTrpIleAsnLeuAspPheLeuOC PheGlnGlyPheLeuTyrLeuPhe
GluArgValLeuTyrValLeuGluPheGlyTyrThrOP ThrLeuCysAsnSerAsnValLeuPheThrPheSer

AlaAsnLysLeuAsnGlnLeuLeuOC OC OP LeuGlnMetLysThrOC LeuLysLeuGluAM AspAlaArg
SerLysGlnIleGluProThrProValIleMetThrThrAsnGluAspIleThrLysValArgIleGlyCysGlu
LysGlnThrAsnOP ThrAsnSerCysAsnAsnAspTyrLysOP ArgHisAsnOC SerAM AsnArgMetArg
AAGCAAACAAATTGAACCAACTCCTGTAATAATGACTACAAATGAAGACATAACTAAAGTTAGAATAGGATGCGA
TTCGTTTGTTTAACTTGGTTGAGGACATTATTACTGATGTTTACTTCTGTATTGATTTCAATCTTATCCTACGCT
LeuLeuCysIleSerGlyValGlyThrIleIleValValPheSerSerMetValLeuThrLeuIleProHisSer
CysValPheGlnValLeuGluGlnLeuLeuSerAM LeuHisLeuCysLeuAM LeuOC PheLeuIleArgPro
AlaPheLeuAsnPheTrpSerArgTyrTyrHisSerCysIlePheValTyrSerPheAsnSerTyrSerAlaLeu

LysAspGlnAsnIleHisAsnGlnOC GluThrGluCysOC ThrTyrThrOC ProGluAsnCysGlnValIle
GluArgProGluHisThrGlnProIleArgAspArgMetLeuAsnIleHisLeuThrArgLysLeuProGlyAsp
GlyLysThrArgThrTyrThrThrAsnLysArgGlnAsnValLysHisThrProAsnGlnLysThrAlaArgOP
GGAAAGACCAGAACATACACAACCAATAAGAGACAGAATGTTAAACATACACCTAACCAGAAAACTGCCAGGTGA
CCTTTCTGGTCTTGTATGTGTTGGTTATTCTCTGTCTTACAATTTGTATGTGGATTGGTCTTTTGACGGTCCACT
SerLeuGlySerCysValCysGlyIleLeuSerLeuIleAsnPheMetCysArgValLeuPheSerGlyProSer
PheValLeuValTyrValValLeuLeuLeuCysPheThrLeuCysValGlyLeuTrpPheValAlaLeuHisAsn
PheSerTrpPheMetCysLeuTrpTyrSerValSerHisOC ValTyrValAM GlySerPheGlnTrpThrIle

TABLE I (cont'd.)

LeuAspPheAM LysLysLeuAsnGlyHisOC TyrValLeuGlyTrpOC ArgLysValThrLysGlnGlnTrp
PheGlyLeuLeuGluGluThrGluTrpProLeuIleCysAlaTrpLeuValLysLysGlyTyrGlnAlaThrMet
PheTrpThrPheArgArgAsnOP MetAlaThrAsnMetCysLeuValGlyLysGluArgLeuProSerAsnAsn
1576 TTTTGGACTTTTAGAAGAAACTGAATGGCCACTAATATGTGCTTGGTTGGTAAAGAAAGGTTACCAAGCAACAAT
AAAACCTGAAAATCTTCTTTGACTTACCGGTGATTATACACGAACCAACCATTTCTTTCCAATGGTTCGTTGTTA
LysProSerLysSerSerValSerHisGlySerIleHisAlaGlnAsnThrPhePheProOC TrpAlaValIle
GlnValLysLeuLeuPheGlnIleAlaValLeuIleHisLysThrProLeuSerLeuAsnGlyLeuLeuLeuPro
LysSerLysOC PhePheSerPheProTrpAM TyrThrSerProGlnTyrLeuPheThrValLeuCysCysHis

LeuAlaIleCysIleIleGlyGluMetTyrLeuIleGlyGlnLysAsnTrpArgSerGlnLysCysIleProGln
AlaSerTyrMetHisHisTrpGlyAsnValProAspTrpSerGluLysLeuGluGluProLysMetHisSerPro
GlyAM LeuTyrAlaSerLeuGlyLysCysThrOP LeuValArgLysIleGlyGlyAlaLysAsnAlaPhePro
1651 GGCTAGCTATATGCATCATTGGGGAAATGTACCTGATTGGTCAGAAAAATTGGAGGAGCCAAAAATGCATTCCCC
CCGATCGATATACGTAGTAACCCCTTTACATGGACTAACCAGTCTTTTTAACCTCCTCGGTTTTTACGTAAGGGG
AlaLeuAM IleCysOP GlnProPheThrGlySerGlnAspSerPheAsnSerSerGlyPheIleCysGluGly
AM SerTyrAlaAspAsnProPheHisValGlnAsnThrLeuPheIleProProAlaLeuPheAlaAsnGlyLeu
SerAlaIleHisMetMetProSerIleTyrArgIleProOP PhePheGlnLeuLeuTrpPheHisMetGlyTrp

OC IleHisGlnGlnThrLeuArgPheProHisGlnOP LysLeuArgGlnArgThrSerThrThrGlnGlnLeu
IleAsnThrProThrAspSerGlnIleSerThrSerValLysThrSerProAlaAspIleAsnTyrAlaAlaThr
AsnLysTyrThrAsnArgLeuSerAspPheHisIleSerGluAsnPheAlaSerGlyHisGlnLeuArgSerAsn
1726 AATAAATACACCAACAGACTCTCAGATTTCCACATCAGTGAAAACTTCGCCAGCGGACATCAACTACGCAGCAAC
TTATTTATGTGGTTGTCTGAGAGTCTAAAGGTGTAGTCACTTTTGAAGCGGTCGCCTGTAGTTGATGCGTCGTTG
IlePheValGlyValSerGluOP IleGluValAspThrPheValGluGlyAlaSerMetLeuAM AlaAlaVal
LeuTyrValLeuLeuSerGluSerLysTrpMetLeuSerPheLysAlaLeuProCysOP SerArgLeuLeuGlu
TyrIleCysTrpCysValArgLeuAsnGlyCysOP HisPheSerArgTrpArgValAspValValCysCysSer

TABLE I (cont'd.)

GlnTyrArgArgThrTrpIleAM LeuAM ProTrpSerArgGlyAlaSerGlnGlnHisGlnLeuSerProThr
ProIleGlnGluAspLeuAspLeuAlaLeuAlaLeuGluProTrpSerGluProThrThrProThrPheThrAsn
SerAsnThrGlyGlyProGlyPheSerPheSerLeuGlyAlaValGluArgAlaAsnAsnThrAsnPheHisGln
1801 TCCAATACAGGAGGACCTGGATTTAGCTTTAGCCTTGGAGCCGTGGAGCGAGCCAACAACACCAACTTTCACCAA
AGGTTATGTCCTCCTGGACCTAAATCGAAATCGGAACCTCGGCACCTCGCTCGGTTGTTGTGGTTGAAAGTGGTT
GlyIleCysSerSerArgSerLysAlaLysAlaLysSerGlyHisLeuSerGlyValValGlyValLysValLeu
LeuValProProGlyProAsnLeuLysLeuArgProAlaThrSerArgAlaLeuLeuValLeuLysOP TrpGly
TrpTyrLeuLeuValGlnIleOC SerOC GlyGlnLeuArgProAlaLeuTrpCysCysTrpSerGluGlyVal

CysThrOC LeuGlnHisArgGlnIleGlnGlnTyrGlyHisGlnValGlnLeuGlyArgLysAM LysProThr
LeuHisLeuThrProThrProProAspSerAlaIleArgThrProSerProThrTrpSerGluIleGluThrAsp
ProAlaLeuAsnSerAsnThrAlaArgPheSerAsnThrAspThrLysSerAsnLeuValGlyAsnArgAsnArg
1876 CCTGCACTTAACTCCAACACCGCCAGATTCAGCAATACGGACACCAAGTCCAACTTGGTCGGAAATAGAAACCGA
GGACGTGAATTGAGGTTGTGGCGGTCTAAGTCGTTATGCCTGTGGTTCAGGTTGAACCAGCCTTTATCTTTGGCT
ArgCysLysValGlyValGlyGlySerGluAlaIleArgValGlyLeuGlyValGlnAspSerIleSerValSer
AlaSerLeuGluLeuValAlaLeuAsnLeuLeuValSerValLeuAspLeuLysThrProPheLeuPheArgCys
GlnValOC SerTrpCysArgTrpIleOP CysTyrProCysTrpThrTrpSerProArgPheTyrPheGlyVal

OC GluProAlaLeuValLysThrValHisHisAsnLysProOP IleArgAM AspGlyValSerCysLysLys
IleArgAlaCysPheGlyGluAsnCysAlaProGlnGlnThrLeuAsnLysValGlyTrpArgLeuLeuGlnLys
HisLysSerLeuLeuTrpOP LysLeuCysThrThrThrAsnLeuGluOC GlyArgMetAlaSerProAlaLys
1951 CATAAGAGCCTGCTTTGGTGAAAACTGTGCACCACAACAAACCTTGAATAAGGTAGGATGGCGTCTCCTGCAAAA
GTATTCTCGGACGAAACCACTTTTGACACGTGGTGTTGTTTGGAACTTATTCCATCCTACCGCAGAGGACGTTTT
MetLeuAlaGlnLysProSerPheGlnAlaGlyCysCysValLysPheLeuThrProHisArgArgArgCysPhe
LeuLeuArgSerGlnHisPheSerHisValValValPheArgSerTyrProLeuIleAlaAspGlyAlaPheLeu
TyrSerGlyAlaLysThrPheValThrCysTrpLeuLeuGlyGlnIleLeuTyrSerProThrGluGlnLeuPhe

TABLE I (cont'd.)

SerLysArgOC GlyAM PheOC GlyGlyGlyGlyHisThrTyrLysThrAsnCysLysOC PhePheTyrIle
GluGlnGluValArgValValLeuArgGlyTrpTrpAlaTyrIleOC AsnOC LeuGlnIleIlePheLeuTyr
ArgAlaArgGlyLysGlySerPheLysGlyValValGlyIleHisIleLysLeuThrAlaAsnAsnPhePheIle
AGAGCAAGAGGTAAGGGTAGTTTTAAGGGGGTGGTGGGCATACATATAAAACTAACTGCAAATAATTTTTTTATA
TCTCGTTCTCCATTCCCATCAAAATTCCCCCACCACCCGTATGTATATTTTGATTGACGTTTATTAAAAAAATAT
SerCysSerThrLeuThrThrLysLeuProHisHisAlaTyrMetTyrPheAM SerCysIleIleLysLysTyr
AlaLeuProLeuProLeuLysLeuProThrThrProMetCysIlePheSerValAlaPheLeuLysLysIleTyr
LeuLeuLeuTyrProTyrAsnOC ProProProProCysValTyrLeuValLeuGlnLeuTyrAsnLysOC Ile

LeuGlnAspOC LeuTyrGlnAspThrAsnThrLeuValGlnGluThrHisAM ThrLysGluAsnGlnLeuIle
IleThrGlyLeuThrLeuProGlyTyrLysTyrLeuGlyProGlyAsnSerLeuAspGlnGlyGluProThrAsn
TyrTyrArgThrAsnSerThrArgIleGlnIleProTrpSerArgLysLeuThrArgProArgArgThrAsnOC
TATTACAGGACTAACTCTACCAGGATACAAATACCTTGGTCCAGGAAACTCACTAGACCAAGGAGAACCAACTAA
ATAATGTCCTGATTGAGATGGTCCTATGTTTATGGAACCAGGTCCTTTGAGTGATCTGGTTCCTCTTGGTTGATT
IleValProSerValArgGlyProTyrLeuTyrArgProGlyProPheGluSerSerTrpProSerGlyValLeu
OC LeuValLeuGluValLeuIleCysIleGlyGlnAspLeuPheSerValLeuGlyLeuLeuValLeuAM Asp
AsnCysSerAM SerAM TrpSerValPheValLysThrTrpSerValOP AM ValLeuSerPheTrpSerIle

HisGlnThrProGlnGlnLysAsnThrThrLysProThrThrAsnThrOC AsnLeuGluLysIleHisThrSer
ProSerAspAlaAlaAlaLysGluHisAspGluAlaTyrAspLysTyrIleLysSerGlyLysAsnProTyrPhe
SerIleArgArgArgSerLysArgThrArgArgSerLeuArgGlnIleHisLysIleTrpLysLysSerIleLeu
TCCATCAGACGCCGCAGCAAAAGAACACGACGAAGCCTACGACAAATACATAAAATCTGGAAAAAATCCATACTT
AGGTAGTCTGCGGCGTCGTTTTCTTGTGCTGCTTCGGATGCTGTTTATGTATTTTAGACCTTTTTTAGGTATGAA
GlyAspSerAlaAlaAlaPheSerCysSerSerAlaAM SerLeuTyrMetPheAspProPhePheGlyTyrLys
MetLeuArgArgLeuLeuLeuValArgArgLeuArgArgCysIleCysLeuIleGlnPhePheAspMetSerArg
TrpOP ValGlyCysCysPhePheValValPheGlyValValPheValTyrPheArgSerPheIleTrpValGlu

TABLE I (cont'd.)

ThrSerGlnGlnLeuMetLysAsnSerOC LysLysLeuAsnThrGlnLysThrThrGluValLysLeuAspIle
TyrPheSerAlaAlaAspGluLysPheIleLysGluThrGluHisAlaLysAspTyrGlyGlyLysIleGlyHis
LeuLeuLeuSerSerOP OP LysIleHisLysArgAsnOP ThrArgLysArgLeuArgArgOC AsnTrpThr
CTACTTCTCAGCAGCTGATGAAAAATTCATAAAAGAAACTGAACACGCAAAAGACTACGGAGGTAAAATTGGACA
GATGAAGAGTCGTCGACTACTTTTTAAGTATTTTCTTTGACTTGTGCGTTTTCTGATGCCTCCATTTTAACCTGT
AM LysGluAlaAlaSerSerPheAsnMetPheSerValSerCysAlaPheSerAM ProProLeuIleProCys
SerArgLeuLeuGlnHisPheIleOP LeuLeuPheGlnValArgLeuLeuSerArgLeuTyrPheGlnValAsn
ValGluOP CysSerIlePhePheGluTyrPhePheSerPheValCysPheValValSerThrPheAsnSerMet

ThrSerSerGluGlnSerValProLeuLeuGlnAsnCysGlnLysGlnThrHisGlnLeuHisLeuAsnAsnGln
TyrPhePheArgAlaLysArgAlaPheAlaProLysLeuSerGluThrAspSerProThrThrSerGlnGlnPro
LeuLeuLeuGlnSerLysAlaCysLeuCysSerLysThrValArgAsnArgLeuThrAsnTyrIleSerThrThr
TTACTTCTTCAGAGCAAAGCGTGCCTTTGCTCCAAAACTGTCAGAAACAGACTCACCAACTACATCTCAACAACC
AATGAAGAAGTCTCGTTTCGCACGGAAACGAGGTTTTGACAGTCTTTGTCTGAGTGGTTGATGTAGAGTTGTTGG
OC LysLysLeuAlaPheArgAlaLysAlaGlyPheSerAspSerValSerGluGlyValValAspOP CysGly
SerArgOP LeuLeuAlaHisArgGlnGluLeuValThrLeuPheLeuSerValLeuAM MetGluValValLeu
ValGluGluSerCysLeuThrGlyLysSerTrpPheGlnOP PheCysValOP TrpSerCysArgLeuLeuTrp

ArgOC GluAspArgArgGluAsnThrGlnGlyLeuAsnHisGlnGluLysAspLeuLeuGlnAspIlePheLeu
GluValArgArgSerProArgLysHisProGlySerLysProProGlyLysArgProAlaProArgHisIlePhe
ArgGlyLysLysIleAlaGluLysThrProArgValOC ThrThrArgLysLysThrCysSerLysThrTyrPhe
AGAGGTAAGAAGATCGCCGAGAAAACACCCAGGGTCTAAACCACCAGGAAAAAGACCTGCTCCAAGACATATTTT
TCTCCATTCTTCTAGCGGCTCTTTTGTGGGTCCCAGATTTGGTGGTCCTTTTTCTGGACGAGGTTCTGTATAAAA
SerThrLeuLeuAspGlyLeuPheCysGlyProAspLeuGlyGlyProPheLeuGlyAlaGlyLeuCysIleLys
ProLeuPheIleAlaSerPheValGlyLeuThrAM ValValLeuPhePheValGlnGluLeuValTyrLysOC
LeuTyrSerSerArgArgSerPheValTrpProArgPheTrpTrpSerPheSerArgSerTrpSerMetAsnLys

## TABLE I (cont'd.)

```
     OC ThrAM LeuLysLysLysLeuLysGlyHisLeuIleGlnThrLeuThrGlnOP ValLysMetTrpAsnAsn
      IleAsnLeuAlaLysLysLysAlaLysGlyThrSerAsnThrAsnSerAsnSerMetSerGlnAsnValGluGln
      TyrLysLeuSerOC LysLysSerOC ArgAspIleOC TyrLysLeuOC LeuAsnGluSerLysCysGlyThr
2476  TATAAACTTAGCTAAAAAAAAAGCTAAAGGGACATCTAATACAAACTCTAACTCAATGAGTCAAAATGTGGAACA
      ATATTTGAATCGATTTTTTTTTCGATTTCCCTGTAGATTATGTTTGAGATTGAGTTACTCAGTTTTACACCTTGT
      IlePheLysAlaLeuPhePheAlaLeuProValAspLeuValPheGluLeuGluIleLeuOP PheThrSerCys
       LeuSerLeuAM PhePheLeuAM LeuSerMetAM TyrLeuSerAM SerLeuSerAspPheHisProValVal
      TyrValOC SerPhePhePheSerPheProCysArgIleCysValArgValOP HisThrLeuIleHisPheLeu

      ThrThrLeuLeuMetGlnAlaLeuAsnCysLeuGlnGlnGluMetAsnLeuGlyValGlyAlaAlaValAlaGly
       HisAsnProIleAsnAlaGlyThrGluLeuSerAlaThrGlyAsnGluSerGlyGlyGlyGlyGlyGlyGlyGly
      ThrGlnProTyrOC CysArgHisOP IleValCysAsnArgLysOP IleTrpGlyTrpGlyArgArgTrpArg
2551  ACACAACCCTATTAATGCAGGCACTGAATTGTCTGCAACAGGAAATGAATCTGGGGGTGGGGGCGGCGGTGGCGG
      TGTGTTGGGATAATTACGTCCGTGACTTAACAGACGTTGTCCTTTACTTAGACCCCCACCCCCGCCGCCACCGCC
      CysLeuGlyIleLeuAlaProValSerAsnAspAlaValProPheSerAspProProProProProProProPro
       CysGlyAM OC HisLeuCysGlnIleThrGlnLeuLeuPheHisIleGlnProHisProArgArgHisArgPro
      ValValArgAsnIleCysAlaSerPheGlnArgCysCysSerIlePheArgProThrProAlaAlaThrAlaPro

      ValGlyValLeuGlyGlyLeuValCysLeuGlnValLeuSerIleIleLysGlnAsnPheAsnThrTrpGlyArg
       GlyArgGlyAlaGlyGlyValGlyValSerThrGlyThrPheAsnAsnGlnThrGluPheGlnTyrLeuGlyGlu
      GlyAM GlyCysTrpGlyGlyTrpCysValTyrArgTyrPheGlnOC SerAsnArgIleSerIleLeuGlyGly
2626  GGGTAGGGGTGCTGGGGGGGGTTGGTGTGTCTACAGGTACTTTCAATAATCAAACAGAATTTCAATACTTGGGGGA
      CCCATCCCCACGACCCCCCCCAACCACACAGATGTCCATGAAAGTTATTAGTTTGTCTTAAAGTTATGAACCCCCT
      ProLeuProAlaProProThrProThrAspValProValLysLeuLeuOP ValSerAsnOP TyrLysProSer
       TyrProHisGlnProProGlnHisThrAM LeuTyrLysOP TyrAspPheLeuIleGluIleSerProProPro
      ThrProThrSerProProAsnThrHisArgCysThrSerGluIleIleLeuCysPheLysLeuValGlnProLeu
```

TABLE I (cont'd.)

AlaTrpLeuGluSerLeuHisThrHisGlnAspSerTyrIleOC IleCysGlnAsnThrLysHisThrLysGlu
GlyLeuValArgIleThrAlaHisAlaSerArgLeuIleHisLeuAsnMetProGluHisGluThrTyrLysArg
GlyLeuGlyAM AsnHisCysThrArgIleLysThrHisThrSerLysTyrAlaArgThrArgAsnIleGlnLys
GGGCTTGGTTAGAATCACTGCACACGCATCAAGACTCATACATCTAAATATGCCAGAACACGAAACATACAAAAG
CCCGAACCAATCTTAGTGACGTGTGCGTAGTTCTGAGTATGTAGATTTATACGGTCTTGTGCTTTGTATGTTTTC
ProLysThrLeuIleValAlaCysAlaAspLeuSerMetCysArgPheIleGlySerCysSerValTyrLeuLeu
SerProOC PheOP GlnValArgMetLeuValOP ValAspLeuTyrAlaLeuValArgPheMetCysPhePhe
AlaGlnAsnSerAspSerCysValCysOP SerGluTyrMetAM IleHisTrpPheValPheCysValPheSer

TyrMetTyrOC IleGlnAsnGlnGlyTrpArgAspLysTrpTyrLysThrMetHisThrHisLysTrpOC His
IleHisValLeuAsnSerGluSerGlyValAlaGlyGlnMetValGlnAspAspAlaHisThrGlnMetValThr
AsnThrCysThrLysPheArgIleArgGlyGlyGlyThrAsnGlyThrArgArgCysThrHisThrAsnGlyAsn
AATACATGTACTAAATTCAGAATCAGGGGTGGCGGGACAAATGGTACAAGACGATGCACACACACAAATGGTAAC
TTATGTACATGATTTAAGTCTTAGTCCCCACCGCCCTGTTTACCATGTTCTGCTACGTGTGTGTGTTTACCATTG
IleCysThrSerPheGluSerAspProThrAlaProCysIleThrCysSerSerAlaCysValCysIleThrVal
ValHisValLeuAsnLeuIleLeuProProProValPheProValLeuArgHisValCysValPheProLeuVal
TyrMetTyrAM IleOP PheOP ProHisArgSerLeuHisTyrLeuValIleCysValCysLeuHisTyrCys

LeuGlyHisOC AM MetLeuThrHisGlyGluCysGlySerIleGlnArgThrGlySerOC TyrProThrThr
ProTrpSerLeuIleAspAlaAsnAlaTrpGlyValTrpPheAsnProAlaAspTrpGlnLeuIleSerAsnAsn
ThrLeuValThrAsnArgCysOC ArgMetGlySerValValGlnSerSerGlyLeuAlaValAsnIleGlnGln
ACCTTGGTCACTAATAGATGCTAACGCATGGGGAGTGTGGTTCAATCCAGCGGACTGGCAGTTAATATCCAACAA
TGGAACCAGTGATTATCTACGATTGCGTACCCCTCACACCAAGTTAGGTCGCCTGACCGTCAATTATAGGTTGTT
GlyGlnAspSerIleSerAlaLeuAlaHisProThrHisAsnLeuGlyAlaSerGlnCysAsnIleAspLeuLeu
LysThrValLeuLeuHisAM ArgMetProLeuThrThrOP AspLeuProSerAlaThrLeuIleTrpCysCys
ArgProOP AM TyrIleSerValCysProSerHisProGluIleTrpArgValProLeuOC TyrGlyValVal

TABLE·I (cont'd.)

```
     OP GlnLysOC ThrAM LeuValLeuAsnLysLysTyrSerMetAM TyrLeuLysGlnLeuGlnAsnGlnGln
     MetThrGluIleAsnLeuValSerPheGluGlnGluIlePheAsnValValLeuLysThrIleThrGluSerAla
     HisAspArgAsnLysLeuSerAM PheOP ThrArgAsnIleGlnCysSerThrOC AsnAsnTyrArgIleSer
2926 CATGACAGAAATAAACTTAGTTAGTTTTGAACAAGAAATATTCAATGTAGTACTTAAAACAATTACAGAATCAGC
     GTACTGTCTTTATTTGAATCAATCAAAACTTGTTCTTTATAAGTTACATCATGAATTTTGTTAATGTCTTAGTCG
     MetValSerIlePheLysThrLeuLysSerCysSerIleAsnLeuThrThrSerLeuValIleValSerAspAla
        SerLeuPheLeuSerLeuOC AsnGlnValLeuPheIleOP HisLeuValOC PheLeuOC LeuIleLeuLeu
     HisCysPheTyrValOC AsnThrLysPheLeuPheTyrGluIleTyrTyrLysPheCysAsnCysPheOP Cys

     ProHisHisGlnProLysTyrIleIleMetIleOC LeuGlnAlaOC TrpSerGlnAM ThrProIleThrHis
        ThrSerProProThrLysIleTyrAsnAsnAspLeuThrAlaSerLeuMetValAlaIleAspThrAsnAsnThr
     AsnLeuThrThrAsnGlnAsnIleOC OC OP SerAsnCysLysLeuAsnGlyArgAsnArgHisGlnOC His
3001 AACCTCACCACCAACCAAAATATATAATAATGATCTAACTGCAAGCTTAATGGTCGCAATAGACACCAATAACAC
     TTGGAGTGGTGGTTGGTTTTATATATTATTACTAGATTGACGTTCGAATTACCAGCGTTATCTGTGGTTATTGTG
     ValGluGlyGlyValLeuIleTyrLeuLeuSerArgValAlaLeuLysIleThrAlaIleSerValLeuLeuVal
        ArgValValLeuTrpPheIleTyrTyrHisAspLeuGlnLeuSerLeuProArgLeuLeuCysTrpTyrCysVal
     GlyOP TrpTrpGlyPheTyrIleIleIleIleAM SerCysAlaOC HisAspCysTyrValGlyIleValCys

     PheHisThrHisGlnGlnHisLeuGluValLysHisLeuValPheIleHisGlyTyrLeuGlnAsnGlnLeuAsn
        LeuProTyrThrProAlaAlaProArgSerGluThrLeuGlyPheTyrProTrpLeuProThrLysProThrGln
     ThrSerIleHisThrSerSerThrAM LysOP AsnThrTrpPheLeuSerMetValThrTyrLysThrAsnSer
3076 ACTTCCATACACACCAGCAGCACCTAGAAGTGAAACACTTGGTTTTTATCCATGGTTACCTACAAAACCAACTCA
     TGAAGGTATGTGTGGTCGTCGTGGATCTTCACTTTGTGAACCAAAAATAGGTACCAATGGATGTTTTGGTTGAGT
     SerGlyTyrValGlyAlaAlaGlyLeuLeuSerValSerProLysOC GlyHisAsnGlyValPheGlyValOP
        GluMetCysValLeuLeuValAM PheHisPheValGlnAsnLysAspMetThrValAM LeuValLeuGluIle
     LysTrpValCysTrpCysCysArgSerThrPheCysLysThrLysIleTrpProOC ArgCysPheTrpSerLeu
```

TABLE I (cont'd.)

ThrAspIleThrTyrHisAlaSerGluThrOC IleHisGlnHisThrLeuAspAsnHisAsnLysOC GlnThr
TyrArgTyrTyrLeuSerCysIleArgAsnLeuAsnProProThrTyrThrGlyGlnSerGlnGlnIleThrAsp
IleGlnIleLeuProIleMetHisGlnLysProLysSerThrAsnIleHisTrpThrIleThrThrAsnAsnArg
3151 ATACAGATATTACCTATCATGCATCAGAAACCTAAATCCACCAACATACACTGGACAATCACAACAAATAACAGA
TATGTCTATAATGGATAGTACGTAGTCTTTGGATTTAGGTGGTTGTATGTGACCTGTTAGTGTTGTTTATTGTCT
TyrLeuTyrOC ArgAspHisMetLeuPheArgPheGlyGlyValTyrValProCysAspCysCysIleValSer
CysIleAsnGlyIleMetCysOP PheGlyLeuAspValLeuMetCysGlnValIleValValPheLeuLeuSer
ValSerIleValAM OP AlaAspSerValAM IleTrpTrpCysValSerSerLeuOP LeuLeuTyrCysVal

GlnTyrLysGlnAspTyrThrValThrLeuCysSerThrGlnAM LysMetGlnTyrGlnPheIlePheOC Glu
SerIleGlnThrGlyLeuHisSerAspIleMetPheTyrThrIleGluAsnAlaValProIleHisLeuLeuArg
LeuAsnThrAsnArgThrThrGlnOP HisTyrValLeuHisAsnArgLysCysSerThrAsnSerSerSerLys
3226 CTCAATACAAACAGGACTACACAGTGACATTATGTTCTACACAATAGAAAATGCAGTACCAATTCATCTTCTAAG
GAGTTATGTTTGTCCTGATGTGTCACTGTAATACAAGATGTGTTATCTTTTACGTCATGGTTAAGTAGAAGATTC
GluIleCysValProSerCysLeuSerMetIleAsnAM ValIleSerPheAlaThrGlyIleOP ArgArgLeu
LeuValPheLeuValValCysHisCysOC ThrArgCysLeuLeuPheHisLeuValLeuGluAspGluLeuPhe
OP TyrLeuCysSerAM ValThrValAsnHisGluValCysTyrPheIleCysTyrTrpAsnMetLysAM Ser

GlnGluMetAsnSerProGlnGluTyrIleThrLeuThrGlnAsnHisOC AsnOC LeuThrHisGlyLysGln
ThrGlyAspGluPheSerThrGlyIleTyrHisPheAspThrLysProLeuLysLeuThrHisSerTrpGlnThr
AsnArgArgOP IleLeuHisArgAsnIleSerLeuOP HisLysThrThrLysIleAsnSerLeuMetAlaAsn
3301 AACAGGAGATGAATTCTCCACAGGAATATATCACTTTGACACAAAACCACTAAAATTAACTCACTCATGGCAAAC
TTGTCCTCTACTTAAGAGGTGTCCTTATATAGTGAAACTGTGTTTTGGTGATTTTAATTGAGTGAGTACCGTTTG
ValProSerSerAsnGluValProIleTyrOP LysSerValPheGlySerPheAsnValOP GluHisCysVal
LeuLeuHisIleArgTrpLeuPheIleAspSerGlnCysLeuValValLeuIleLeuGluSerMetAlaPheLeu
CysSerIlePheGluGlyCysSerTyrIleValLysValCysPheTrpAM PheOC SerValOP ProLeuCys

## TABLE I (cont'd.)

```
    ThrAspLeuAM AspCysLeuGlnAsnTyrOC LeuAsnLeuProGlnLysGluThrAsnSerGlnGluHisTyr
    AsnArgSerLeuGlyLeuProProLysLeuLeuThrGluProThrThrGluGlyAspGlnLeuProGlyThrLeu
    LysGlnIleSerArgThrAlaSerLysThrThrAsnOP ThrTyrHisArgArgArgProThrProArgAsnThr
3376 AAACAGATCTCTAGGACTGCCTCCAAAACTACTAACTGAACCTACCACAGAAGGAGACCAACTCCCAGGAACACT
     TTTGTCTAGAGATCCTGACGGAGGTTTTGATGATTGACTTGGATGGTGTCTTCCTCTGGTTGAGGGTCCTTGTGA
     PheLeuAspArgProSerGlyGlyPheSerSerValSerGlyValValSerProSerTrpSerGlyProValSer
      CysIleGluLeuValAlaGluLeuValValLeuGlnValAM TrpLeuLeuLeuGlyValGlyLeuPheValVal
     ValSerArgAM SerGlnArgTrpPheAM AM SerPheArgGlyCysPheSerValLeuGluTrpSerCysAM

    GlnGlnLeuThrGlnGluLysValIleThrLysGlnLeuIleIleAlaThrGlnLysGlnGlnGlnLeuGly
    ProAlaAlaAsnThrArgLysGlyTyrHisGlnThrIleAsnAsnSerTyrThrGluAlaThrAlaIleArg
    ThrSerSerOC HisLysLysArgLeuSerProAsnAsnOC OC AM LeuHisArgSerAsnSerAsnAM Ala
3451 ACCAGCAGCTAACACAAGAAAAGGTTATCACCAAACAATTAATAATAGCTACACAGAAGCAACAGCAATTAGGCC
     TGGTCGTCGATTGTGTTCTTTTCCAATAGTGGTTTGTTAATTATTATCGATGTGTCTTCGTTGTCGTTAATCCGG
     GlyAlaAlaLeuValLeuPheProOC OP TrpValIleLeuLeuLeuAM ValSerAlaValAlaIleLeuGly
      LeuLeuAM CysLeuPheLeuAsnAspGlyPheLeuOC TyrTyrSerCysLeuLeuLeuLeuLeuOC Ala
     TrpCysSerValCysSerPheThrIleValLeuCysAsnIleIleAlaValCysPheCysCysCysAsnPro
```

0117063

- 30 -

0117063

Table I sets out in capital letters the continuous sequence of base pairs of porcine parvovirus RF DNA, as identified, commencing with approximately the 300th base of the 5 kb PPV genome (i.e., commencing at about the Pstl restriction site to the left in Figure 1) and through approximately 3400 bases thereafter (i.e., to the HaeIII restriction site to the right in Figure 1). Flanking the DNA sequence are six possible amino acid sequences into which the DNA could be translated, depending upon the reading frame. Stop codons are designated as OC, or OP, or AM. More specifically, the three amino acid sequences above the DNA sequence represent three left-to-right translations of the upper DNA strand and the sequences below represent the three possible right-to-left translations.

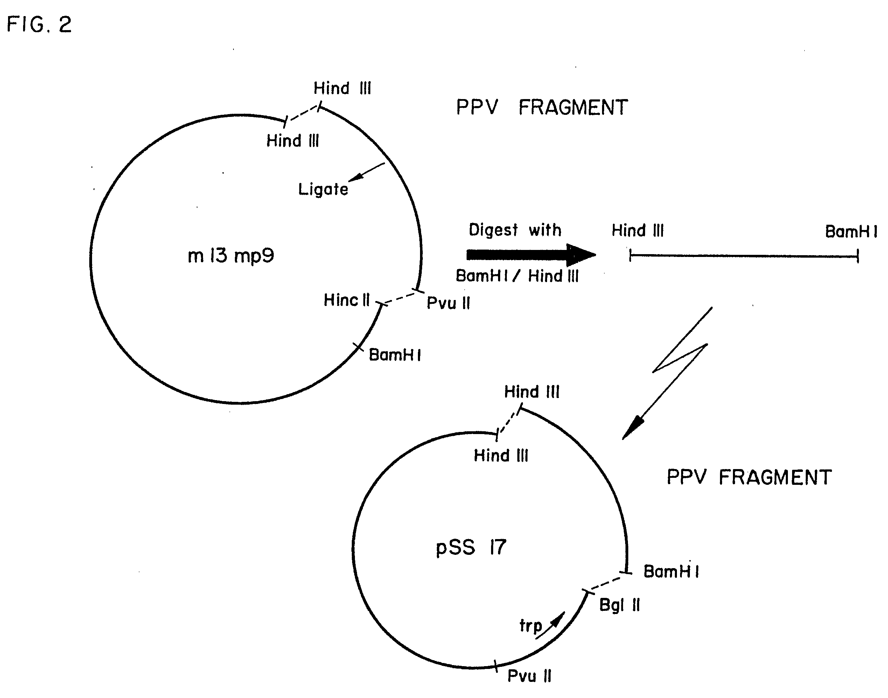

Later sequence confirmation of the continuous sequence of base pairs of porcine parvovirus RF DNA disclosed in Figure 2 of U.S. Patent Application Serial No. 459,203, revealed that four bases originally present in Figure 2 sequence were not confirmed and are hence absent from the sequence in Table I herein; 130 bases were omitted from the original sequence and have been inserted herein; and 9 bases were incorrectly identified in Figure 2 of the parent application and are corrected in Table I herein.

The specific corrections are noted in the sequence of Table I as follows:

(1) At reference No. 199 of the sequence of Table I, the parent application indicated the presence of an A/T pair, which was not confirmed upon sequence verification;

(2) At reference Nos 273 through 401 of the sequence of Table I is a sequence of 129 bases omitted from Figure 2 of the parent application;

(3) At reference No 488 of the sequence of Table I, the parent application indicated the presence of a G/C pair, which was not confirmed upon sequence
verification;

(4) At reference Nos 494-496 of the sequence
of Table I, Figure 2 of the parent application indicated
the presence of three T/A pairs, which upon sequence
verification were shown to be A/T pairs;

(5) At reference No. 520 of the sequence of
Table I, Serial No. 459,203 indicated the presence of an
additional A/T pair whose presence was not confirmed
upon sequence verification;

(6) At reference No. 557 of the sequence of
Table I, Serial No. 459,203 failed to indicate the presence of a G/C pair revealed upon sequence verification;

(7) At reference No. 702 of the sequence of
Table I, Serial No. 459,203 indicated the presence of a
T/A pair, which was not confirmed upon sequence verification; and

(8) At reference Nos. 816 through 821 of the
sequence of Table I, Serial No. 459,203 indicated the
presence of the following sequence

5'-CCAAAA-3'
3'-GGTTTT-5'

which, upon sequence verification was determined to be

5'-TTCCCC-3'
3'- AAGGGG-5'

Examination of the entire sequence of Table I
in all possible reading frames reveals the presence of
one major coding phase in the direction predicted by
hybridization data, left-to-right in Figure 1.  This
open frame extends from positions 0.82 through (and
possibly beyond) 3.85 kb with the exception of a small
region near 2.2 kb.  A computer search of the sequence
also revealed the existence of several canonical RNA
splicing junction sequences.  Left-hand splice sites
(AGGTA) are found at 7 positions and right-hand sites
[TT (any) CAG] at 2 positions.  Since these sequences are only 5 unique nucleotides in length, they would be expected to occur randomly about once every 1000 nucleotides; therefore their presence only indicates the possibility of splicing events occurring. Splicing is known to occur, however, in a closely related parvovirus, H1, wherein S1 nuclease mapping experiments indicated that 4.7, 3.0 and 2.8 kb viral transcripts were the product of the splicing of a common 2.6 kb segment to smaller non-continguous segments. See, Green, et al., Cell, 17, 967 (1979). Preliminary results suggest an analogous situation in PPV. Hybridization of total polyA+RNA from PPV infected cells to the negative strand genomic DNA produced 2 major S1 resistant duplex bands on a neutral agarose gel with lengths 2.8 and 2.6 kb and a less intense band at 4.7 kb. In addition, some hybrids viewed in the electron microscope appear to be the result of DNA hybridization to spliced RNA, although such evidence is not conclusive.

Table II sets out in capital letters the DNA sequence of Table I, together with the coded amino acid sequence having the highest probability of being duplicative of the translational result of porcine parvovirus DNA-directed viral protein manufacture.

TABLE II

```
    CysSerMetAlaAlaGlyAsnThrTyrSerGluGluValLeuLysAlaThrAsnTrpLeu
             10        20        30         40        50        60
      Bbvl     Bbvl                    Mboll Rsal     Alul
 2   TGCAGCATGGCAGCGGGAAACACTTACTCGGAAGAGGTACTAAAAGCTACCAACTGGCTT
     ACGTCGTACCGTCGCCCTTTGTGAATGAGCCTTCTCCATGATTTTCGATGGTTGACCGAA
     Fnu4hl   Fnu4hl                         r  Mnl
```

```
    GlnAspAsnAlaGlnLysGluAlaPheSerTyrValPheLysThrGlnLysValAsnLeu
             10        20        30         40        50        60
 62  CAAGATAATGCTCAAAAAGAAGCATTCTCTTATGTATTTAAAACACAAAAAGTCAATCTA
     GTTCTATTACGAGTTTTTCTTCGTAAGAGAATACATAAATTTTGTGTTTTTCAGTTAGAT
```

```
    AsnGlyLysGluIleAlaTrpAsnAsnTyrAsnLysAspThrThrAspAlaGluMetIle
             10        20        30        40        50        60
                   Asull                           SfaNl
122  AATGGAAAAGAAATTGCTTGGAATAACTACAACAAAGATACAACAGATGCGGAAATGATA
     TTACCTTTTCTTTAACGAACCTTATTGATGTTGTTTCTATGTTGTCTACGCCTTTACTAT
```

TABLE II (cont'd.)

```
      AsnLeuGlnArgGlyAlaGluThrSerTrpAspGlnAlaThrAspMetGluTrpGluSer
              10        20        30        40        50          60
             .Mnl                  AsulEcorll                   Hinfl
182   AACCTACAAAGAGGAGCAGAAACATCATGGGACCAGGCAACAGACATGGAATGGGAATCA
      TTGGATGTTTCTCCTCGTCTTTGTAGTACCCTGGTCCGTTGTCTGTACCTTACCCTTAGT
                                       Avall


      GluIleAspSerLeuThrLysArgGlnValLeuIlePheAspSerLeuValLysLysCys
              10        20        30        40        50          60
           Taql    Mnl             Rrul          Hinfl
242   GAAATCGACAGCCTCACAAAACGGCAAGTACTGATTTTTGACTCTCTTGTTAAAAAATGT
      CTTTAGCTGTCGGAGTGTTTTGCCGTTCATGACTAAAAACTGAGAGAACAATTTTTTACA
                                       Rsal


      LeuPheGluGlyIleLeuGlnLysAsnLeuSerProSerAspCysTyrTrpPheLeuGln
              10        20        30        40        50          60
                                 Ddel
302   CTCTTTGAAGGTATATTGCAAAAGAACCTAAGTCCAAGTGACTGCTACTGGTTCTTACAG
      GAGAAACTTCCATATAACGTTTTCTTGGATTCAGGTTCACTGACGATGACCAAGAATGTC


      HisGluHisGlyGlnAspThrGlyTyrHisCysHisValLeuLeuGlyGlyLysGlyLeu
              10        20        30        40        50          60
                                          Rsal
362   CATGAACATGGTCAAGATACTGGCTATCACTGCCATGTACTACTAGGTGGAAAAGGCTTA
      GTACTTGTACCAGTTCTATGACCGATAGTGACGGTACATGATGATCCACCTTTTCCGAAT
```

TABLE II (cont'd.)

GlnGlnAlaMetGlyLysTrpLeuArgLysGlnLeuAsnAsnLeuTrpSerArgTrpLeu
   10   20   30   40   50   60

CAACAAGCAATGGGAAAATGGTTACGAAAACAATTAAACAATTTATGGAGTAGATGGTTG
GTTGTTCGTTACCCTTTTACCAATGCTTTTGTTAATTTGTTAAATACCTCATCTACCAAC


AsnAsnGlnCysLysValProLeuThrProValGluArgIleLysLeuArgGluLeuAla
   10   20   30   40   50   60
     RsaI

AATAATCAATGCAAAGTACCTCTAACACCAGTTGAAAGAATAAAATTAAGGGAATTAGCA
TTATTAGTTACGTTTCATGGAGATTGTGGTCAACTTTCTTATTTTAATTCCCTTAATCGT
       MnI


GluAspGlyGluTrpValSerLeuLeuThrTyrThrHisLysGlnThrLysLysGlnTyr
   10   20   30   40   50   60
MnI  Hph

GAGGATGGTGAGTGGGTATCGCTACTAACCTACACTCACAAACAAACTAAAAAACAATAT
CTCCTACCACTCACCCATAGCGATGATTGGATGTGAGTGTTTGTTTGATTTTTTGTTATA


ThrLysMetThrHisPheGlyAsnMetIleAlaTyrTyrPheLeuAsnLysLysArgLys
   10   20   30   40   50   60
  HinfI  AsuII

ACAAAAATGACTCATTTTGGAAATATGATTGCTTACTACTTCCTAAATAAAAAAAGAAAG
TGTTTTTACTGAGTAAAACCTTTATACTAACGAATGATGAAGGATTTATTTTTTTCTTTC

- 35 -

0117063

TABLE II (cont'd.)

```
      ThrThrGluArgGluHisGlyTyrTyrLeuSerSerAspSerGlyPheMetThrAsnPhe
            10        20        30        40        50        60
                                  Ddel Ddel
662   ACAACTGAAAGAGAGCATGGATATTATCTCAGCTCAGATTCTGGCTTCATGACAAATTTC
      TGTTGACTTTCTCTCGTACCTATAATAGAGTCGAGTCTAAGACGGAAGTACTGTTTAAAG
                                    Alul  Hinfl


      LeuLysGluGlyGluArgHisLeuValSerHisLeuPheThrGluAlaAsnLysProGlu
            10        20        30        40        50        60
                              Ddel      Hph
722   TTAAAAGAAGGCGAGAGACACTTAGTCAGTCACCTATTTACTGAAGCAAATAAACCTGAA
      AATTTTCTTCCGCTCTCTGTGAATCAGTCAGTGGATAAATGACTTCGTTTATTTGGACTT


      ThrValGluThrThrValThrThrAlaGlnGluValProArgGlyArgIleGlnThrLys
            10        20        30        40        50        60
                                  Alul          Aval
782   ACTGTGGAAACAACGGTTACTACAGCTCAGGAAGTTCCCCGAGGCAGAATACAAACAAAA
      TGACACCTTTGTTGCCAATGATGTCGAGTCCTTCAAGGGGCTCCGTCTTATGTTTGTTTT
                                  Ddel          Mnl
```

TABLE II (cont'd.)

```
        LysGluValSerIleLysCysThrIleArgAspLeuValAsnLysArgCysThrSerIle
             10        20        30        40        50        60
                                                              Rsal
  842  AAAGAAGTAAGCATAAAATGCACAATAAGAGACTTGGTTAATAAAAGATGTACTAGCATA
       TTTCTTCATTCGTATTTTACGTGTTATTCTCTGAACCAATTATTTTCTACATGATCGTAT
```

```
        GluGlyTrpSerMetThrAspProAspSerTyrIleGluMetMetAlaGlnThrGlyGly
             10        20        30        40        50        60
                  XhoII                                  HpaII
  902  GAAGGCTGGAGTATGACAGATCCAGACAGTTATATAGAAATGATGGCTCAAACCGGAGGA
       CTTCCGACCTCATACTGTCTAGGTCTGTCAATATATCTTTACTACCGAGTTTGGCCTCCT
                       MboI                                   MnI
```

```
        GluAsnLeuIleLysAsnThrLeuGluIleThrThrLeuThrLeuAlaArgThrLysThr
             10        20        30        40        50        60
  962  GAAAATTTAATCAAAAATACACTAGAAATAACAACTCTTACTCTAGCAAGAACAAAAACA
       CTTTTAAATTAGTTTTTATGTGATCTTTATTGTTGAGAATGAGATCGTTCTTGTTTTTGT
```

```
        AlaTyrAspLeuIleLeuGluLysAlaLysProSerMetLeuProThrPheAsnIleSer
             10        20        30        40        50        60
                                           SphI
 1022  GCATATGACTTAATACTTGAAAAGGCAAAACCAAGCATGCTACCAACATTTAATATTAGC
       CGTATACTGAATTATGAACTTTTCCGTTTTGGTTCGTACGATGGTTGTAAATTATAATCG
```

TABLE II (cont'd.)

```
        AsnThrArgThrCysLysIlePheSerMetHisAsnTrpAsnTyrIleLysValCysHis
             10     I    20        30        40        50        60
                                 Sph1         AsuII
    1082 AATACAAGAACATGTAAAATATTCAGCATGCACAATTGGAACTACATTAAAGTCTGCCAT
         TTATGTTCTTGTACATTTTATAAGTCGTACGTGTTAACCTTGATGTAATTTCAGACGGTA
```

```
        AlaIleThrCysValLeuLysGlnThrArgArgLysLysLysTyrAsnSerIleSerCys
             10        20        30        40        50        60
               Rsa1                    Mnl
    1142 GCTATAACTTGTGTACTGAAACAGACAAGGAGGAAAAAGAAATACAATTCTATTTCATGT
         CGATATTGAACACATGACTTTGTCTGTTCCTCCTTTTTCTTTATGTTAAGATAAAGTACA
```

```
        HisGlyProAlaSerThrGlyLysSerIleIleAlaGlnHisIleAlaAsnLeuValGly
             10        20        30        40        50        60
           ,Asul                                         Ddel
    1202 CATGGGCCAGCATCAACAGGAAAAAGTATAATTGCTCAACACATTGCAAACTTAGTTGGT
         GTACCCGGTCGTAGTTGTCCTTTTTCATATTAACGAGTTGTGTAACGTTTGAATCAACCA
           HaeIII
```

TABLE II (cont'd.)

```
AsnValGlyCysTyrAsnAlaAlaAsnValAsnPheProPheAsnAspCysThrAsnLys
     10        20        30        40        50        60
                    Fnu4hl                        RsaI
1262 AATGTTGGTTGCTACAATGCAGCCAATGTGAACTTTCCATTTAATGACTGTACAAATAAA
     TTACAACCAACGATGTTACGTCGGTTACACTTGAAAGGTAAATTACTGACATGTTTATTT
                    BbvI
```

```
AsnLeuIleTrpIleGluGluAlaGlyAsnPheSerAsnGlnValAsnGlnPheLysAla
     10        20        30        40        50        60
              MboII
1322 AACTTAATATGGATTGAAGAAGCAGGAAACTTCTCTAACCAAGTAAACCAATTCAAAGCC
     TTGAATTATACCTAACTTCTTCGTCCTTTGAAGAGATTGGTTCATTTGGTTAAGTTTCGG
```

```
IleCysSerGlyGlnThrIleArgIleAspGlnLysGlyLysGlySerLysGlnIleGlu
     10        20        30        40        50        60
1382 ATATGTTCAGGTCAAACAATTAGAATTGACCAAAAAGGTAAAGGAAGCAAACAAATTGAA
     TATACAAGTCCAGTTTGTTAATCTTAACTGGTTTTTCCATTTCCTTCGTTTGTTTAACTT
```

```
ProThrProValIleMetThrThrAsnGluAspIleThrLysValArgIleGlyCysGlu
     10        20        30        40        50        60
                              MboII                   SfaNlMn
1442 CCAACTCCTGTAATAATGACTACAAATGAAGACATAACTAAAGTTAGAATAGGATGCGAG
     GGTTGAGGACATTATTACTGATGTTTACTTCTGTATTGATTTCAATCTTATCCTACGCTC
```

TABLE II (cont'd.)

```
GluArgProGluHisThrGlnProIleArgAspArgMetLeuAsnIleHisLeuThrArg
        10        20        30        40    .   50        60
1
1502 GAAAGACCAGAACATACACAACCAATAAGAGACAGAATGTTAAACATACACCTAACCAGA
     CTTTCTGGTCTTGTATGTGTTGGTTATTCTCTGTCTTACAATTTGTATGTGGATTGGTCT
```

```
LysLeuProGlyAspPheGlyLeuLeuGluGluThrGluTrpProLeuIleCysAlaTrp
        10        20        30        40        50        60
        Ecorll                Mboll          HaelHaelll
1562 AAACTGCCAGGTGATTTTGGACTTTTAGAAGAAACTGAATGGCCACTAATATGTGCTTGG
     TTTGACGGTCCACTAAAACCTGAAAATCTTCTTTGACTTACCGGTGATTATACACGAACC
        Hph                                  Ball
```

```
LeuValLysLysGlyTyrGlnAlaThrMetAlaSerTyrMetHisHisTrpGlyAsnVal
        10        20        30        40        50        60
        Bstell              .  Alul  Avalll              Rs
1622 TTGGTAAAGAAAGGTTACCAAGCAACAATGGCTAGCTATATGCATCATTGGGGAAATGTA
     AACCATTTCTTTCCAATGGTTCGTTGTTACCGATCGATATACGTAGTAACCCCTTTACAT
```

- 40 -

0117063

TABLE II (cont'd.)

```
    ProAspTrpSerGluLysLeuGluGluProLysMetHisSerProIleAsnThrProThr
            10        20        30        40        50        60
    al                Mnl           AvaIII
1682 CCTGATTGGTCAGAAAAATTGGAGGAGCCAAAAATGCATTCCCCAATAAATACACCAACA
     GGACTAACCAGTCTTTTTAACCTCCTCGGTTTTTACGTAAGGGGTTATTTATGTGGTTGT


    AspSerGlnIleSerThrSerValLysThrSerProAlaAspIleAsnTyrAlaAlaThr
            10        20        30        40        50        60
    Hinfl                                             Bbvl
1742 GACTCTCAGATTTCCACATCAGTGAAAACTTCGCCAGCGGACATCAACTACGCAGCAACT
     CTGAGAGTCTAAAGGTGTAGTCACTTTTGAAGCGGTCGCCTGTAGTTGATGCGTCGTTGA
     Ddel                                              Fnu4hl


    ProIleGlnGluAspLeuAspLeuAlaLeuAlaLeuGluProTrpSerGluProThrThr
            10        20        30        40        50        60
         MnlAvall        Alul
1802 CCAATACAGGAGGACCTGGATTTAGCTTTAGCCTTGGAGCCGTGGAGCGAGCCAACAACA
     GGTTATGTCCTCCTGGACCTAAATCGAAATCGGAACCTCGGCACCTCGCTCGGTTGTTGT
            AsulEcorll


    ProThrPheThrAsnLeuHisLeuThrProThrProProAspSerAlaIleArgThrPro
            10        20        30        40        50        60
    Hph                                 Hinfl
1862 CCAACTTTCACCAACCTGCACTTAACTCCAACACCGCCAGATTCAGCAATACGGACACCA
     GGTTGAAAGTGGTTGGACGTGAATTGAGGTTGTGGCGGTCTAAGTCGTTATGCCTGTGGT
```

TABLE II (cont'd.)

```
          SerProThrTrpSerGluIleGluThrAspIleArgAlaCysPheGlyGluAsnCysAla
                 10          20          30          40      ·  50          60
                                                                Hph         HgiA
    1922 AGTCCAACTTGGTCGGAAATAGAAACCGACATAAGAGCCTGCTTTGGTGAAAACTGTGCA
         TCAGGTTGAACCAGCCTTTATCTTTGGCTGTATTCTCGGACGAAACCACTTTTGACACGT


          ProGlnGlnThrLeuAsnLysValGlyTrpArgLeuLeuGlnLysGluGlnGluValArg
                 10          20          30          40          50          60
                                            Acyl                    Mnl
         1
    1982 CCACAACAAACCTTGAATAAGGTAGGATGGCGTCTCCTGCAAAAAGAGCAAGAGGTAAGG
         GGTGTTGTTTGGAACTTATTCCATCCTACCGCAGAGGACGTTTTTCTCGTTCTCCATTCC


          ValValLeuArgGlyTrpTrpAlaTyrIleOC
                   10          20          30          40          50          60
    2042 GTAGTTTTAAGGGGGTGGTGGGCATACATATAAAACTAACTGCAAATAATTTTTTTATAT
         CATCAAAATTCCCCCACCACCCGTATGTATATTTTGATTGACGTTTATTAAAAAAATATA
```

TABLE II (cont'd.)

GlyLeuThrLeuProGlyTyrLysTyrLeuGlyProGlyAsnSerLeuAspGln
     10      20      30      40      50      60
             Ecorll           AvallEcorll

2102  ATTACAGGACTAACTCTACCAGGATACAAATACCTTGGTCCAGGAAACTCACTAGACCAA
       TAATGTCCTGATTGAGATGGTCCTATGTTTATGGAACCAGGTCCTTTGAGTGATCTGGTT
                                     Asul


GlyGluProThrAsnProSerAspAlaAlaAlaLysGluHisAspGluAlaTyrAspLys
     10      20      30      40      50      60
             AcylFnu4hlBbvl

2162  GGAGAACCAACTAATCCATCAGACGCCGCAGCAAAAGAACACGACGAAGCCTACGACAAA
       CCTCTTGGTTGATTAGGTAGTCTGCGGCGTCGTTTTCTTGTGCTGCTTCGGATGCTGTTT
              Hgal   Fnu4hl


TyrIleLysSerGlyLysAsnProTyrPheTyrPheSerAlaAlaAspGluLysPheIle
     10      20      30      40      50      60
                         DdelFnu4hlAlul

2222  TACATAAAATCTGGAAAAAATCCATACTTCTACTTCTCAGCAGCTGATGAAAAATTCATA
       ATGTATTTTAGACCTTTTTTAGGTATGAAGATGAAGAGTCGTCGACTACTTTTTAAGTAT
                                 BbvlPvull


LysGluThrGluHisAlaLysAspTyrGlyGlyLysIleGlyHisTyrPhePheArgAla
     10      20      30      40      50      60
             Mnl               Mboll

2282  AAAGAAACTGAACACGCAAAAGACTACGGAGGTAAAATTGGACATTACTTCTTCAGAGCA
       TTTCTTTGACTTGTGCGTTTTCTGATGCCTCCATTTTAACCTGTAATGAAGAAGTCTCGT

TABLE II (cont'd.)

```
LysArgAlaPheAlaProLysLeuSerGluThrAspSerProThrThrSerGlnGlnPro
      10        20 ·       30        40        50        60
                                   Hinfl        ·
AAGCGTGCCTTTGCTCCAAAACTGTCAGAAACAGACTCACCAACTACATCTCAACAACCA
TTCGCACGGAAACGAGGTTTTGACAGTCTTTGTCTGAGTGGTTGATGTAGAGTTGTTGGT
                                   Hph
```

```
GluValArgArgSerProArgLysHisProGlySerLysProProGlyLysArgProAla
      10        20        30        40        50        60
 Mnl     MbollGdill       Ecorll          Ecorll
GAGGTAAGAAGATCGCCGAGAAAACACCCAGGGTCTAAACCACCAGGAAAAAGACCTGCT
CTCCATTCTTCTAGCGGCTCTTTTGTGGGTCCCAGATTTGGTGGTCCTTTTTCTGGACGA
         Mbol
```

```
ProArgHisIlePheIleAsnLeuAlaLysLysLysAlaLysGlyThrSerAsnThrAsn
      10        20        30        40        50        60
                  Ddel            Alul
CCAAGACATATTTTTATAAACTTAGCTAAAAAAAAAGCTAAAGGGACATCTAATACAAAC
GGTTCTGTATAAAAATATTTGAATCGATTTTTTTTTCGATTTCCCTGTAGATTATGTTTG
                  Alul
```

TABLE II (cont'd.)

SerAsnSerMetSerGlnAsnValGluGlnHisAsnProIleAsnAlaGlyThrGluLeu
        10          20          30          40          50          60
            Hinfl
2522   TCTAACTCAATGAGTCAAAATGTGGAACAACACAACCCTATTAATGCAGGCACTGAATTG
       AGATTGAGTTACTCAGTTTTACACCTTGTTGTGTTGGGATAATTACGTCCGTGACTTAAC


SerAlaThrGlyAsnGluSerGlyGlyGlyGlyGlyGlyGlyGlyGlyArgGlyAlaGly
        10          20          30          40          50          60
            Hinfl               Fnu4hl
2582   TCTGCAACAGGAAATGAATCTGGGGGGTGGGGGCGGCGGTGGCGGGGGTAGGGGTGCTGGG
       AGACGTTGTCCTTTACTTAGACCCCCACCCCCGCCGCCACCGCCCCCATCCCCACGACCC


GlyValGlyValSerThrGlyThrPheAsnAsnGlnThrGluPheGlnTyrLeuGlyGlu
        10          20          30          40          50          60
          Accl      Rsal                                        Mn
2642   GGGGTTGGTGTGTCTACAGGTACTTTCAATAATCAAACAGAATTTCAATACTTGGGGGAG
       CCCCAACCACACAGATGTCCATGAAAGTTATTAGTTTGTCTTAAAGTTATGAACCCCCTC

TABLE II (cont'd.)

```
GlyLeuValArgIleThrAlaHisAlaSerArgLeuIleHisLeuAsnMetProGluHis
        10          20          30          40          50          60
      Hinfl                          Hinfl
2702  GGCTTGGTTAGAATCACTGCACACGCATCAAGACTCATACATCTAAATATGCCAGAACAC
      CCGAACCAATCTTAGTGACGTGTGCGTAGTTCTGAGTATGTAGATTTATACGGTCTTGTG
```

```
GluThrTyrLysArgIleHisValLeuAsnSerGluSerGlyValAlaGlyGlnMetVal
        10          20          30          40          50          60
                  Rsal          Hinfl                              Rs
2762  GAAACATACAAAAGAATACATGTACTAAATTCAGAATCAGGGGTGGCGGGACAAATGGTA
      CTTTGTATGTTTTCTTATGTACATGATTTAAGTCTTAGTCCCCACCGCCCTGTTACCAT
```

```
GlnAspAspAlaHisThrGlnMetValThrProTrpSerLeuIleAspAlaAsnAlaTrp
        10          20          30          40          50          60
al    SfaNl                                              SfaNl
2822  CAAGACGATGCACACACACAAATGGTAACACCTTGGTCACTAATAGATGCTAACGCATGG
      GTTCTGCTACGTGTGTGTGTTTACCATTGTGGAACCAGTGATTATCTACGATTGCGTACC
```

```
GlyValTrpPheAsnProAlaAspTrpGlnLeuIleSerAsnAsnMetThrGluIleAsn
        10          20          30          40          50          60
Ddel
2882  GGAGTGTGGTTCAATCCAGCGGACTGGCAGTTAATATCCAACAACATGACAGAAATAAAC
      CCTCACACCAAGTTAGGTCGCCTGACCGTCAATTATAGGTTGTTGTACTGTCTTTATTTG
```

- 46 -

0117063

TABLE II (cont'd:)

```
LeuValSerPheGluGlnGluIlePheAsnValValLeuLysThrIleThrGluSerAla
        10        20        30        40         50        60
                                            Rrul              Hinfl
2942  TTAGTTAGTTTTGAACAAGAAATATTCAATGTAGTACTTAAAACAATTACAGAATCAGCA
      AATCAATCAAAACTTGTTCTTTATAAGTTACATCATGAATTTTGTTAATGTCTTAGTCGT
                                        Rsal
```

```
ThrSerProProThrLysIleTyrAsnAsnAspLeuThrAlaSerLeuMetValAlaIle
        10        20        30        40         50        60
      Mnl                         Mbol        Hindlll
3002  ACCTCACCACCAACCAAAATATATAATAATGATCTAACTGCAAGCTTAATGGTCGCAATA
      TGGAGTGGTGGTTGGTTTTATATATTATTACTAGATTGACGTTCGAATTACCAGCGTTAT
      Hph                                        Alul
```

```
AspThrAsnAsnThrLeuProTyrThrProAlaAlaProArgSerGluThrLeuGlyPhe
        10        20        30        40        50        60
                                Fnu4hl
3062  GACACCAATAACACACTTCCATACACACCAGCAGCACCTAGAAGTGAAACACTTGGTTTT
      CTGTGGTTATTGTGTGAAGGTATGTGTGGTCGTCGTGGATCTTCACTTTGTGAACCAAAA
                                      Bbvl
```

```
TyrProTrpLeuProThrLysProThrGlnTyrArgTyrTyrLeuSerCysIleArgAsn
        10        20        30        40        50        60
      Bstell                                       Avalll
3122  TATCCATGGTTACCTACAAAACCAACTCAATACAGATATTACCTATCATGCATCAGAAAC
      ATAGGTACCAATGGATGTTTTGGTTGAGTTATGTCTATAATGGATAGTACGTAGTCTTTG
```

TABLE II (cont'd.)

```
    LeuAsnProProThrTyrThrGlyGlnSerGlnGlnIleThrAspSerIleGlnThrGly
         10           20           30           40           50           60
                                                          Hinfl
3182  CTAAATCCACCAACATACACTGGACAATCACAACAAATAACAGACTCAATACAAACAGGA ·
      GATTTAGGTGGTTGTATGTGACCTGTTAGTGTTGTTTATTGTCTGAGTTATGTTTGTCCT   ·


    LeuHisSerAspIleMetPheTyrThrIleGluAsnAlaValProIleHisLeuLeuArg
         10           20           30           40           50           60
                                                    Rsal        MboII
3242  CTACACAGTGACATTATGTTCTACACAATAGAAAATGCAGTACCAATTCATCTTCTAAGA ·
      GATGTGTCACTGTAATACAAGATGTGTTATCTTTTACGTCATGGTTAAGTAGAAGATTCT
                                                                  Ddel


    ThrGlyAspGluPheSerThrGlyIleTyrHisPheAspThrLysProLeuLysLeuThr
      ,    10           20           30           40           50           60
              Ecorl
3302  ACAGGAGATGAATTCTCCACAGGAATATATCACTTTGACACAAAACCACTAAAATTAACT
      TGTCCTCTACTTAAGAGGTGTCCTTATATAGTGAAACTGTGTTTTGGTGATTTTAATTGA
```

TABLE II (cont'd.)

```
     HisSerTrpGlnThrAsnArgSerLeuGlyLeuProProLysLeuLeuThrGluProThr
          10        20        30        40        50        60
                    XhollMbol          Mnl
3362  CACTCATGGCAAACAAACAGATCTCTAGGACTGCCTCCAAAAACTACTAACTGAACCTACC
      GTGAGTACCGTTTGTTTGTCTAGAGATCCTGACGGAGGTTTTGATGATTGACTTGGATGG
                      Bgl11
```

```
     ThrGluGlyAspGlnLeuProGlyThrLeuProAlaAlaAsnThrArgLysGlyTyrHis
          10        20        30        40        50        60
                    Ecorll          BbvlAlul              Hph
3422  ACAGAAGGAGACCAACTCCCAGGAACACTACCAGCAGCTAACACAAGAAAAGGTTATCAC
      TGTCTTCCTCTGGTTGAGGGTCCTTGTGATGGTCGTCGATTGTGTTCTTTTCCAATAGTG
                                      Fnu4hl
```

```
     GlnThrIleAsnAsnSerTyrThrGluAlaThrAlaIleArg
          10        20        30        40
                    Alul                    Hae
3482  CAAACAATTAATAATAGCTACACAGAAGCAACAGCAATTAGGCC
      GTTTGTTAATTATTATCGATGTGTCTTCGTTGTCGTTAATCCGG
```

(Note that the amino acid sequence corresponds to that shown in the middle sequence above the DNA strand in Table I. Correspondingly, the DNA sequence of Table II lacks the initial base pair C/G illustrated in Table I.) Table II also illustrates computer deduced recognition sites for cleavage by common restriction endonucleases.

The sequence disclosed in Table II corresponds to Figure 3 of U.S. Patent Application Serial No. 459,203 except for the changes indicated above for the sequence of Table I. However, because the base changes, deletions and insertions made to the sequence of Figures 2 and 3 of Serial No. 459,203 appearing in Tables I and II, respectively, totalled a number of bases divisible by three, the changes did not effect the reading frame of Table II, which is identical to that of Figure 3. Only the reference numbers were changed to reflect the information obtained upon later sequence verification. Additionally, the last change made to the sequence of Figures 2 and 3 of the parent application occurred at reference numbers 816 through 821 of Tables I and II, which is prior to the coding regions of the polypeptides employed in the following examples.

Table III sets out in capital letters the DNA sequence of porcine parvovirus RF DNA, as identified, commencing with approximately the 4200th base of the 5 kb PPV genome (i.e., commencing at about the 2nd Bgl II restriction site to the right in Figure 1) and through approximately 400 bases thereafter (i.e., to the 2nd Acc I restriction site to the right in Figure 1).

Illustrated with the DNA sequence is an amino acid sequence revealed by an open reading frame and most likely to duplicate that present in the carboxy terminus of one or more PPV capsid proteins. This amino acid sequence shows substantial homology to the carboxy terminus proposed by Astrell, et al. (1983) supra.

## TABLE III

AspLeuLysProArgLeuHisValThrAlaProPheValCysLys

GATCTAAAACCTAGACTACATGTTACAGCTCCATTTGTTTGTAAA
CTAGATTTTGGATCTGATGTACAATGTCGAGGTAAACAAACATTT

AsnAsnProProGlyGlnLeuPheValLysIleAlaProAsnLeu

AACAATCCACCAGGACAACTATTTGTAAAAATAGCACCAAACCTA
TTGTTAGGTGGTCCTGTTGATAAACATTTTTATCGTGGTTTGGAT

ThrAspAspPheAsnAlaAspSerProGlnGlnProArgIleIle

ACAGATGATTTCAATGCTGACTCTCCTCAACAACCTAGAATAATA
TGTCTACTAAAGTTACGACTGAGAGGAGTTGTTGGATCTTATTAT

ThrTyrSerAsnPheTrpTrpLysGlyThrLeuThrPheThrAla

ACTTATTCAAACTTTTGGTGGAAAGCAACACTAACATTCACAGCA
TGAATAAGTTTGAAAACCACCTTTCGTTGTGATTGTAAGTGTCGT

LysMetArgSerSerAsnMetTrpAsnProSerGlnGlnHisThr

AAAATGAGATCCAGTAATATGTGGAACCCTAGTCAACAACACACA
TTTTACTCTAGGTCATTATACACCTTGGGATCAGTTGTTGTGTGT

ThrThrAlaGluAsnIleGlyAsnTyrIleProThrAsnIleGly

ACAACAGCAGAAAACATTGGTAACTATATTCCTACAAATATTGGT
TGTTGTCGTCTTTTGTAACCATTGATATAAGGATGTTTATAACCA

GlyIleArgMetPheProGluTyrSerGlnLeuIleProIleLys

GGCATAAGAATGTTTCCAGAATATTCACAACTTATACCAATAAAA
CCGTATTCTTACAAAGGTCTTATAAGTGTTGAATATGGTTATTTT

LeuTyrAM

TTATACTAGAAATAACTCTGTAAATAAAAACTCAGTTACTTGGTT
AATATGATCTTTATTGAGACATTTATTTTTGAGTCAATGAACCAA

AATCATGTACTACTATCATTGTATAC
TTAGTACATGATTGTAGTAACATATG

The information set out in Tables I to III makes possible, for the first time, the cloning of genomic fragments from PPV genome coding regions in the correct sense and translational frame allowing for microbial expression of polypeptides which duplicate amino acid sequences present in parvovirus proteins. The sequence data also makes possible the manufacture (by, e.g., chemical synthesis) of selected, relatively low molecular weight polypeptides which, by virtue of their amino acid sequence similarities to parvovirus, can be expected to share biological properties of parvovirus proteins such as antigenicity and immunogenicity.

Also provided by the information set out in Tables I to III is a basis for prediction of relatively highly hydrophilic regions within selected parvovirus-duplicative polypeptides to be manufactured, which regions are likely to incorporate antigenic determinants. See, e.g., Hopp, et al. P.N.A.S., 78, 3824 (1981) and Kyte, et al., J.Mol.Biol., 157, 105 (1982).

By way of illustration, the amino acid sequence of Table II was subjected to computerized analysis for hydrophilic and hydrophobic regions according to the Hopp, et al. "method" by means of a program and designated PEP Reference §6.7 made available by Intelligenetics, Inc., 124 University Avenue, Palo Alto, California. Refering to Table III, the amino acid sequence consisting of the series,

Thr-Arg-Arg-Lys-Lys-Lys-Tyr,

coded for by base pairs numbered 1166 through 1186, comprises the most highly hydrophilic region of the total sequence. This polypeptide sequence, or a sequence which substantially duplicates it, would be expected to be of substantial immunological significance. It appears, however, that the DNA sequence coding for this polypeptide sequence resides in an intron region not likely to be translated into a capsid protein.

- 53 -

0117063

The following example relates to the construction of a representative DNA vector according to the present invention.

EXAMPLE 5

As graphically depicted in Figure 2, a DNA vector pSS17 was constructed to contain a fragment of the PPV genome under the control of a trp promoter sequence. The initial procedure involved excision of a PPV genomic fragment of approximately 780 base pairs by digestion of the 5kb PPV monomer replicative form with restriction endonuclease enzymes PvuII and HindIII. PvuII cleaves at a site indicated at position 2.56 on the 5 kb restriction map illustrated in Figure 1. The specific sequence involved in PvuII cleavage is illustrated within the DNA sequence adjacent reference numeral 2262 of Table II. HindIII cleaves at a site indicated at position 3.35 on Figure 1 and specifically illustrated adjacent reference numeral 3050 of Table II.

The fragment was inserted into M13mp9 between the HincIII and HindIII restriction endonuclease enzyme recognition sites. The DNA was then digested with HindIII and BamHI to yield a fragment duplicating the original PPV fragment plus a few nucleotides of M13. A pBR322-derived plasmid p41 retaining the gene for ampicillin resistance and containing the "trp" operon of E. coli operatively associated with the entire trpE structural gene and a portion of the trpD structural gene [Cheng, et al. Gene, 14, 121-130 (1981)] was digested with BglII and HindIII. The aforementioned BglII cut digestion occurred at a first site in the p41 vector (1398 bases) downstream of a PvuII site. The HindIII/BamHI PPV fragment was then inserted into digested p41, forming plasmid pSS17 which includes a fusion protein comprising the first 320 amino acids of trpE and 260 amino acids coded for by PPV DNA.

Transformation of the plasmid pSS17 into E. coli strain HB101 yielded drug resistant colonies which were screened for the presence of derived PPV sequences by hybridization to a probe radiolabelled by nick translation of total genomic PPV. The presence of PPV sequences was also conformed by restriction endonuclease mapping of the insert using AccI, PvuII and HindIII.

Bacterial lysates prepared from E. coli strain AM7$\overline{\text{P}}^{\text{r}}$ cells transformed with plasmid pSS17 isolated from the above-transformed HB101 strain did contained detectable but not appreciable amounts of the projected trpE/PPV fusion polypeptide.

The following Example relates to the construction of another DNA vector according to the present invention for the expression of the projected trp/PPV fusion polypeptide of Example 5.


EXAMPLE 6


Further manipulations were performed in an attempt to generate an expression vector which would provide for high levels of E. coli expression of the desired fusion protein. These manipulations involved use of a plasmid which is the subject of co-owned, co-pending U.S. Patent Application Serial No. 521,964, by Morris, entitled "DNA Vector". The disclosures of said application are specifically incorporated by reference herein. Briefly noted, plasmid pCFM414 (A.T.C.C. 40076), employed in the construction, includes a temperature-sensitive mutation in the copy control region. Upon transformation with this vector, host cell cultures grown at 34·C will have a low number of copies of the plasmid. The plasmid copy number increases fifty-fold (i.e., "runs away") within the host cells upon elevation of the culture temperature above 34·C. Growth at 37·C will ordinarily be lethal to the cells.

The specific manipulations involved in construction of a vector using A.T.C.C. 40076 were as follows. Plasmid pSS17 was digested with HindIII and PvuII to generate a fragment including the PPV structural gene and the associated trp promoter/operator. Plasmid pCFM414 was digested with HindIII and HpaI and the large fragment was isolated. The HindIII/PvuII blunt-ended fragment containing the trp/PPV gene was then ligated with the large HindIII/HpaI fragment of pCFM414 to generate plasmid p/H6. While this construction retained an intact HindIII recognition site, ligation of the PvuII site with the HpaI terminus did not restore either recognition site.

E. coli strain Am70$^I$ cells transformed with plasmid p/H6 produced measurable amounts of the trpE/PPV fusion polypeptide estimated to be on the order of 8 mg/OD liter of cells.

The following Example relates to the construction of another DNA vector employing another PPV fragment according to the present invention.

EXAMPLE 7

A second fragment of approximately 350 base pairs of the PPV genome was excised from the 5 kb PPV monomer replicative form by double digestion with restriction endonuclease enzyme Sau3A. Sau3A cleaves at two sites indicated at positions 3.33 and 3.68 on the 5 kb restriction endonuclease map of Figure 1. The specific sequence involved in Sau3A cleavage is illustrated within the DNA sequence adjacent reference numerals 3034 and 3383 of Table II.

Following amplification in M13mp8, this Sau3A fragment was inserted into a pBR-322-derived plasmid, pINTγ-trpI7 previously digested with BamHI. Plasmid pINTγ-trpI7 is disclosed in co-owned co-pending U.S. Patent Application Serial No. 483,451 by Alton, et al.,

entitled "The Manufacture and Expression of Large Structural Genes". The disclosures of said reference are specifically incorporated by reference herein. Briefly noted, pINTγtrpI-7 was constructed in the following manner.

Plasmid PVvI (purchased from Stanford University, Palo Alto, California) was digested with PvuII and using standard procedures an EcoRI recognition site was inserted in the plasmid at a PvuII site. Copies of this hybrid were then digested with EcoRI and HpaI to provide a 245 base pair sequence including a portion of the trp promoter/operator region. By standard procedures, a DNA sequence subunit of the sequence for human interferon, IF-4, was added to the HpaI site in order to incorporate the remaining 37 base pairs of the complete trp translational initiation signal and bases providing codons for the initial four amino acids of immune interferon (Cys-Tyr-Cys-Gln). The resulting assembly was then inserted into pINT3, a pBR322 derivative, which had been digested with EcoRI and BamHI to yield plasmid pINTγ-trpI7.

When the Sau3A/Sau3A PPV fragment is inserted into BamHI-digested pINTγ-trpI7, the ligation of the complementary sticky ends of Sau3A and BamHI did not restorethe BamHI restriction site. The resulting plasmid containing the PPV genome behind a trp promoter and four amino acids of γ interferon, was designated S/S3.

E. coli strain AM70$^T$ cells transformed with plasmid pS/S3 produced measurable amounts of the γ IFN/PPV fusion polypeptide, estimated to be on the order of 5 mg/OD litre of cells.

The following example relates to the construction of yet another DNA vector employing a third fragment of the PPV genome according to the present invention.

EXAMPLE 8

A third fragment of approximately 479 base pairs of the PPV genome was excised from the 5 kb PPV monomer replicative form by digestion with restriction endonuclease enzymes HindIII and HaeIII. HindIII cleaves at a site indicated at position 3.35 of Figure 1 and specifically illustrated adjacent reference numeral 3050 of Table II. HaeIII cleaves at a site indicated at position 3.82 on Figure 1 and the specific sequence involved in HaeIII cleavage is illustrated within the DNA sequence adjacent reference numeral 3524 of Table II.

The fragment was inserted for amplification into M13mp9 between the HincII and HindIII restriction endonuclease enyzme recognition sites, thereby eliminating both the HincII and HaeIII sites. The DNA is then digested with EcoRI to yield a fragment duplicating the original PPV fragment plus a few nucleotides of M13 at the former HaeIII terminus of the fragment and minus 269 PPV nucleotides located between the EcoRI and HindIII sites of the PPV fragment.

Plasmid p41 described previously in Example 5 and containing the β-galactosidase gene was also cleaved with EcoRI and the PPV fragment inserted therein, resulting in plasmid pE/H2.5 containing a β-gal/PPV fusion gene.

E. coli strain AM7$\overline{\phi}^I$ cells transformed with plasmid pE/H2.5 produced measurable amounts of the fusion polypeptide in bacterial lysate, estimated to be on the order of 15 mg/OD liter.

The following example relates to the development of a synthetic PPV polypeptide according to the present invention.

EXAMPLE 9

The amino acid sequence of the region of the 5 kb PPV monomer replicative form which provided the coding regions for the above-described PPV fragments (i.e., from approximately position 2.5 to 5.0) was subjected to computerized analysis for hydrophilic and hydrophobic regions according to the Hopp, et al. "method" by means of a program and designated PEP Reference §6.7 made available by Intelligenetics, Inc., 124 University Avenue, Palo Alto, Cal.

Two peptide sequences had a hydrophilicity profile that predicted a possible surface orientation in the native PPV molecule:

NH$_2$-Asn-Leu-Ala-Lys-Lys-Lys-Ala-Lys-Gly-Thr-Ser-Asn-Thr-Asn-Ser-COOH

and

NH$_2$-Thr-Thr-Ser-Gln-Gln-Pro-Glu-Val-Arg-Arg-Ser-Pro-Arg-Lys-COOH.

Table IV below indicates the hydropathicity (-) and hydrophilicity (+) of the amino acid sequence so analyzed.

## TABLE IV

| Table II Reference Nos. 2309 through 2602 Amino Acid | Hydropathicity/ Hydrophilicity Values |
|---|---|
| Gly | +8 |
| Gly | +3 |
| Lys | -2 |
| Ile | +1 |
| Gly | -2 |
| His | -7 |
| Tyr | -17 |
| Phe | -8 |
| Phe | -9 |
| Arg | -3 |
| Ala | +6 |
| Lys | +10 |
| Arg | +10 |
| Ala | +3 |
| Phe | +4 |
| Ala | +4 |
| Pro | -4 |
| Lys | -2 |
| Leu | +7 |
| Ser | +7 |
| Glu | +13 |
| Thr | +8 |
| Asp | +11 |
| Ser | +10 |
| Pro | +3 |
| Thr | +5 |
| Thr | -- |
| Ser | -- |
| Gln | -- |
| Gln | +6 |

## TABLE IV (cont'd.)

| Table II Reference Nos. 2309 through 2602 Amino Acid | Hydropathicity/ Hydrophilicity Values |
|---|---|
| Pro | +4 |
| Glu | ÷8 |
| Val | +14 |
| Arg | +14 |
| Arg | +14 |
| Ser | +14 |
| Pro | +22 |
| Arg | +16 |
| Lys | ÷10 |
| His | +10 |
| Pro | +10 |
| Gly | +10 |
| Ser | ÷5 |
| Lys | ÷6 |
| Pro | +6 |
| Pro | +11 |
| Gly | +16 |
| Lys | +11 |
| Arg | +10 |
| Pro | +10 |
| Ala | +15 |
| Pro | +9 |
| Arg | -- |
| His | -4 |
| Ile | -6 |
| Phe | -6 |
| Ile | -15 |
| Asn | -15 |
| Leu | -6 |
| Ala | +3 |

TABLE IV (cont'd.)

| Table II Reference Nos. 2309 through 2602 Amino Acid | Hydropathicity/ Hydrophilicity Values |
|---|---|
| Lys | +11 |
| Lys | +20 |
| Ala | +21 |
| .Lys | +14 |
| Gly | +9 |
| Thr | +4 |
| Ser | +4 |
| Asn | -- |
| Thr | -- |
| Asn | +1 |
| Ser | +1 |
| Asn | -1 |
| Ser | -- |
| Met | -- |
| Ser | -- |
| Gln | -3 |
| Asn | +1 |
| Val | +4 |
| Glu | +2 |
| Gln | +2 |
| His | +2 |
| Asn | +2 |
| Pro | -3 |
| Ile | -4 |
| Asn | -3 |
| Ala | -4 |
| Gly | -- |

## TABLE IV (cont'd.)

| Table II Reference Nos. 2309 through 2602 Amino Acid | Hydropathicity/ Hydrophilicity Values |
|---|---|
| Thr | -- |
| Glu | +1 |
| Leu | +1 |
| Ser | -- |
| Ala | +1 |
| Thr | -4 |
| Gly | +4 |
| Asn | +4 |
| Glu | +6 |
| Ser | +9 |

A second analysis was performed on the first peptide from Ref Nos. 2480 to 2524 to determine its hydropathicity when it was separated from the adjacent amino acid sequences. This peptide, designated TJ-1, proved to be highly hydrophilic as shown in Table V below and was therefore selected for synthesis.

## TABLE V

| Amino Acid | Hydropathicity/ Hydrophilicity Values |
|---|---|
| Asn | -11 |
| Leu | +3 |
| Ala | +13 |
| Lys | +19 |
| Lys | +17 |
| Lys | +31 |
| Ala | +32 |
| Lys | +22 |
| Gly | +15 |
| Thr | +7 |
| Ser | +7 |
| Asn | -- |
| Thr | -- |
| Asn | +2 |
| Ser | +1 |

- 64 -  0117063

Peptide TJ-1 was chemically synthesized according to the Merrifield resin-bound procedure described in Stewart, et al., _Solid Phase Peptide Synthesis_, (W. H. Freeman, San Francisco, 1969). Briefly put, peptides were constructed by means of a series of amino acid additions to an initial, column-bound residue selected to form the carboxy terminal residue of the peptide. THE selected carboxyl terminal amino acid is coupled to the polystyrene resin as a BOC-protected amino acid. All subsequent amino acid additions are carried out with dicyclohexylcarbodiimide using the appropriate BOC amino acid with side chain protecting groups as follows: threonine as O-benzyl threonine; lysine as 2-chlorocarbobenzoxy lysine; asparagine as xanthyl asparagine; and, serine as O-benzyl serine. Finished protected peptides are cleaved from the resin with simultaneous deprotection using anhydrous HF. The Peptide was purified by a combination of chromatography on Sephadex G25 and preparative thin layer chromatography. The desired product was isolated as a stable lyophilized white powder. Composition was determined by amino acid analysis after HCl digestion as described in "Protein Sequence Determination" page 197 (S. Needleman, ed., Sprinbger-Verlag, 1975). Sequence verification was performed by automated amino acid analysis. Purified products migrated as a single spot on TLC. An Rf value of o.23 was determined for TJ-1 with a pH 4-4.5 solvent comprising butanol, acetic acid, water and pyridine (15:3:12:10).

The following example relates to the use of the PPV fusion polypeptides and synthetic peptide described above in producing antisera to PPV.

## EXAMPLE 10

Isolates from AM7$\bar{\underline{P}}^{r}$ cultures containing plasmids E/H 2.5, S/S 3 and PH6 as described above, a column-purified isolate of PH6 and two preparations of the synthetic peptide TJ-1 were employed to produce antisera for use in plate binding and neutralization assays. One of the TJ-1 preparations was covalently cross-linked to keyhole limpet hemocyanin (KLH) using gluteraldehyde according to the procedure of Baron, et al., Cell, 28, pp. 395-404 (1982) while the other was unlinked.

Antisera were specifically produced by injecting rabbits with each isolate or peptide preparation according to the following protocol. Initially, pre-immune sera was collected from each animal. The first inoculation contained the amount of antigen indicated in Table VI below with 0.5 ml of Freund's complete adjuvant. Succeeding inoculations contained the antigens and 0.25 ml of Freund's incomplete adjuvant. The animals were bled on days 28 and 63.

## TABLE VI

### Amounts of Inoculum (µg)

| Antigens: | E/H 2.5 | PH6 | S/S 3 | (Linked to KLH) TJ-1 | (Column-Purified PH6 | Unlinked TJ-1 |
|---|---|---|---|---|---|---|
| DAYS |  |  |  |  |  |  |
| 1 | 150 | 150 | 250 | 250 | 250 | 250 |
| 7 | 75 | 75 | 125 | 125 | 250 | 250 |
| 21 | 75 | 75 | 125 | 125 | 250 | 250 |
| 35 | 75 | 75 | 125 | 125 | 250 | 250 |
| 56 | 75 | 75 | 125 | 125 | 250 | 250 |

The following example relates to tests performed on the antisera obtained with PPV fusion polypeptides and the synthetic peptide described above to determine their immunogenicity.

## EXAMPLE 11

### A.  Plate Binding Assay

A plate binding assay was performed according to the procedures of Engvall, et al., J.Immunol., 109, 129-135 (1972) and Von Weemen, et al. FEBS Letters, 24, 77-81 (1972) testing the antisera produced in Example 10 against the antigen used to produce them and against PPV.  In addition, anti-PPV serum from an infected pig was tested against each antigen.  The titers shown in Table VII below are the serum dilutions at which the binding between antibodies in the antisera and the indicated antigens fell to one half the saturation level.

### TABLE VII

| Antibody | Immunizing Antigen | PPV |
|---|---|---|
| Anti-E/H 2.5 | $7.5 \times 10^3$ | $1.5 \times 10^3$ |
| Anti-TJl(KLH linked) | $1.6 \times 10^5$ | $3.0 \times 10^4$ |
| Anti-P/H6 | $6.25 \times 10^3$ | $\leq 10^2$ |
| Anti-PPV | E/H2.5 $1.0 \times 10^2$ | $1.38 \times 10^4$ |
|  | TJl $2.0 \times 10^2$ |  |
|  | P/H6 $4.0 \times 10^2$ |  |

These results indicate that the antisera raised to the synthetic TJl was capable of binding its own antigen as well as native PPV in infected pig serum when compared to the other fusion peptides.

B. Plaque Reduction Assay

The antisera were further tested in a plaque reduction assay, according to the procedures of "A Manual of Basic Virological Techniques," (Rovozzo, et al.) pp. 94-103 (Prentice-Hall, Englewood Cliffs, N.J. 1973) to determine their ability to neutralize PPV infection. Results for Anti-TJl are reported in Table VIII below. Only TJ-1 antisera proved to be neutralizing in the assay, having a titer several orders of magnitude lower than anti-PPV serum from natural infection. ST cells were plated at a density of $1.5 \times 10^6$ cells per 60 mm dish and the assay was performed after six hours.

TABLE VIII

Plaque Reduction By Anti-TJ-1

| Dilutions Sera | Plate Nos. | Preimmune Sera | Immune |
|---|---|---|---|
| 1:25 | 25 | 178 | 116 |
| 1:100 | 25 | 113 | 202 |
| 1:25 | 26 | 128 | 20 |
| 1:100 | 26 | 213 | 153 |
| 1:25 | 27 | 124 | 110 |
| 1:100 | 27 | 168 | 140 |
| 1:25 | 28 | 92 | 79 |
| 1:100 | 28 | 166 | 145 |

These results indicate that antisera raised to the synthetic peptide TJ-1 was capable of neutralizing the infectivity of PPV by causing a consistent reduction in plaques formed in a serum culture infected with PPV.

Polypeptides of the invention, including the fusion polypeptides of the foregoing Examples 6 and 7 and the synthetic peptides of Example 8 are expected to display significant value as immunogenic constituents of vaccine compositions for administration to animals susceptible to parvovirus infection and to provoke protective immune responses in vaccinated animals. Vaccine compositions according to the invention therefore comprise immunologically effective quantities of polypeptides of the invention in combination with immunologically acceptable diluent, adjuvants and carriers. Like the prior art killed and modified parvovirus vaccines, they are expected to be optimally useful when administered parenterally.

While polypeptides made available according to the inventon are at least in part duplicative of amino acid sequences extant in porcine parvovirus proteins, expected homologies between PPV proteins and those of parvoviruses specific for other host species are expected to make the polypeptides useful in development of vaccinal immunity against infection by other parvovirus species other than PPV (e.g., canine or bovine parvovirus). Owing to their structural similarity to native parvovirus proteins, the polypeptides of the invention are also expected to be useful as reagents in assays directed to quantitative determination of anti-parvovirus antibodies in fluid samples such as animal serum.

While the presently preferred method for manufacture of immunologically active polypeptides of the invention is through microbial expression of DNA sequences which are wholly or partially duplicative of sequences extant in the parvovirus genome, it will be understood that manufactured DNA sequences which are the "translational equivalents" of parvovirus genome sequences may also be employed in microbial production processes. It is within the comprehension of the invention that the specific and immunologically active PPV polypeptides be manufactured by way of microbial expression of manufactured DNA sequences coding for the identical amino acid sequences as PPV genomic fragments but by means of alternate codons which may be the subject of transcriptional or translational preference in organism selected for production. (See, e.g., U.S. Letters Patent No. 4,356,270.)

It will be clear to those skilled in the art that the present invention comprehends chemical or biological synthesis employing well-known methods of polypeptide assembly of immunologically active polypeptides of substantial length (such as coded for by the 780 base pair sequence of PPV DNA in pSS17) as well as shorter sequences which may possess significant immunological activity. As a general rule, biologically or chemically synthesized polyeptides of the invention may include 6 or more amino acids.

Numerous modifications and variations in the invention as described in the above-detailed description are expected to be apparent to those skilled in the arts of immunology and the use of recombinant methods for the arts of immunology and the use of recombinant methods for the manufacture of polypeptides. As one example, the polypeptide which is designated as the subject of expression of the PPV DNA sequence of plasmid pH/6 will be a fusion peptide. As such it may be cleaved from association with E. coli trpE amino acids before being put to use or it may be used as an immunologically active agent in the form of a fusion protein of PPV polypeptide and a non-viral polypeptide. Alternately, of course, the PPV derived DNA of pH/6 may be incorporated into any suitable transformation vector having a promoter/regulator sequence other than the "trp" operon and the vector may be designed to permit direct expression of the PPV DNA directed sequence.

As another example, polypeptides of the invention or monoclonal or polyclonal antibodies thereto are expected to useful (either with or without detectable labels such as radiolabels) in immunoassays such as RIAs and ELISAs for the detection of porcine and other species parvovirus antigens and/or antibodies in fluid samples. Similarly, DNA sequences provided by the invention (e.g., PPV DNA sequences provided in amplified form as inserts in autonomously replicating viral or circular plasmid DNA vectors) are expected to provide useful sources of single-stranded DNA for incorporation in DNA/DNA hybridization procedures for the detection of porcine and other species parvovirus DNA in tissue samples.

In view of the above, only such limitations should be placed upon the invention as appear in the appended claims.

The features disclosed in the foregoing description, in the following claims and/or in the accompanying drawings may, both separately and in any combination thereof, be material for realising the invention in diverse forms thereof.

0117063

CLAIMS

1. An immunologically active polypeptide useful in development of vaccinal immunity against parvovirus infection and characterized by being the product of the microbial expression of a DNA sequence wholly or partially duplicative of a DNA sequence extant in a parvovirus genome or of a translational equivalent thereof.

2. A polypeptide product according to claim 1 wherein the DNA sequence microbially expressed is duplicative of a sequence present in a porcine parvovirus genome.

3. A polypeptide product according to claim 1 which is the product of joint microbial expression with a non-viral polypeptide in a single translational event.

4. A polypeptide product according to claim 1 which is the product of microbial expression of a DNA sequence wholly or partially duplicative of that set forth in Tables II or III or of a translational equivalent thereof.

5. A polypeptide product according to claim 1 and further characterized by the presence of multiple hydrophilic amino acids in series.

6. A chemically synthesized immunologically active polypeptide useful in the development of vaccinal immunity against parvovirus infection and characterized by the presence of amino acids in a series which is wholly or partially duplicative of the sequence of amino acids set forth in Tables II or III.

7. A vaccine composition for use in provoking
an immune response protective against parvovirus infection comprising an immunologically effective amount of a
polypeptide according to claim 1 or 6 and an immunologically acceptable diluent, adjuvant or carrier.

8. A DNA transformation vector suitable for
use in securing the expression of immunologically significant polypeptides and characterized by the presence of
nucleotide base pairs in a sequence duplicative of that
extant in a parvovirus genome or the translational
equivalent thereof.

9. A method for the production of immunologically active polypeptides comprising: transforming a
suitable microbial host cell with a vector according to
claim 8; growing cells so transformed under suitable
nutrient conditions and isolating the desired polypeptide therefrom.

10. A DNA sequence wholly or partially duplicative of the DNA sequences set forth in Table II or III.

11. A reagent material comprising a DNA
sequence according to claim 10 and a detectable label.

12. A reagent material according to claim 11
wherein the detectable label is a radiolabel.

13. A reagent material comprising a polypeptide
according to claim 1 or 6 or an antibody thereto and a
detectable label.

14. A reagent material according to claim 13
wherein the detectable label is a radiolabel.

FIG. I

LENGTH IN KILOBASE PAIRS

DIRECTION OF TRANSLATION →

BASE-SEQUENCED REGIONS

FIG. 2

PPV FRAGMENT

Hind III

Hind III

Ligate

m 13 mp9

Digest with

BamHI / Hind III

Hind III        BamHI

Hinc II        Pvu II

BamHI

Hind III

Hind III

PPV FRAGMENT

pSS 17

BamHI

Bgl II

trp

Pvu II

**European Patent Office**

**EUROPEAN SEARCH REPORT**

| | DOCUMENTS CONSIDERED TO BE RELEVANT | | EP 84300312.0 |

| Category | Citation of document with indication, where appropriate, of relevant passages | Relevant to claim | CLASSIFICATION OF THE APPLICATION (Int. Cl. 3) |
|---|---|---|---|
| A,D | PROCEEDINGS OF THE NATIONAL ACADEMY OF SCIENCES OF THE UNITED STATES OF AMERICA, vol. 76, no. 11, November 1979, Baltimore, Md. (USA) <br><br> D. REVIE et al "Covalent association of Protein with replicative form DNA of parvovirus H-1" pages 5539-5543 <br><br> * Abstract * <br><br> -- | 1,2 | C 12 N   15/00 <br> C 12 P   21/04 <br> C 12 P   21/00 <br> C 12 N    7/00 <br> C 07 C  103/52 <br> A 61 K   39/23 |
| A,D | JOURNAL OF VIROLOGY, vol. 41, no. 3, March 1982, (Washington, USA) <br><br> S.L. RHODE et al. "DNA Sequence of the 5'Terminus Containing the Replication Origin of Parvovirus Replicative Form DNA" pages 990-999 <br><br> * Page 990 * <br><br> -- | 1,8 | |
| A,P | WO – A1 – 83/02 393 (ANIMAL VACCINE RESEARCH CORPORATION) <br><br> * Abstract * <br><br> ---- | 1 | **TECHNICAL FIELDS SEARCHED (Int. Cl. 3)** <br><br> C 12 N <br> C 12 P <br> C 07 C  103/00 <br> A 61 K |

The present search report has been drawn up for all claims

| Place of search | Date of completion of the search | Examiner |
|---|---|---|
| VIENNA | 09-04-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82